(19) Europäisches Patentamt
European Patent Office
Office européen des brevets

(11) EP 0 729 341 B1

(12) **EUROPÄISCHE PATENTSCHRIFT**

(45) Veröffentlichungstag und Bekanntmachung des Hinweises auf die Patenterteilung:
**05.04.2000 Patentblatt 2000/14**

(21) Anmeldenummer: **95933376.6**

(22) Anmeldetag: **15.09.1995**

(51) Int Cl.7: **A61K 6/10**

(86) Internationale Anmeldenummer:
**PCT/EP95/03638**

(87) Internationale Veröffentlichungsnummer:
**WO 96/08230 (21.03.1996 Gazette 1996/13)**

# (54) HYDROPHILIERTE ZAHNABDRUCKMASSEN

HYDROPHILIZED DENTAL IMPRESSION COMPOUNDS

COMPOSITIONS HYDROPHILES POUR EMPREINTE DENTAIRE

(84) Benannte Vertragsstaaten:
**AT BE CH DE ES FR GB IT LI SE**

(30) Priorität: **16.09.1994 DE 4433139**

(43) Veröffentlichungstag der Anmeldung:
**04.09.1996 Patentblatt 1996/36**

(73) Patentinhaber: **ESPE Dental AG**
**82229 Seefeld (DE)**

(72) Erfinder:
- **ZECH, Joachim**
  **D-82229 Hechendorf (DE)**
- **WANEK, Erich**
  **D-86916 Kaufering (DE)**
- **LECHNER, Günther**
  **D-82237 Wörthsee (DE)**
- **BISSINGER, Peter**
  **D-86343 Königsbrunn (DE)**

(74) Vertreter: **Abitz, Walter, Dr.-Ing. et al**
**Patentanwälte Abitz & Partner**
**Postfach 86 01 09**
**81628 München (DE)**

(56) Entgegenhaltungen:
**EP-A- 0 231 420**
**EP-A- 0 398 745**
**EP-A- 0 480 238**
**DE-C- 4 320 920**
**GB-A- 1 520 421**
**GB-A- 2 203 152**
**US-A- 4 657 959**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 729 341 B1

## Beschreibung

Die vorliegende Erfindung betrifft durch spezielle Zusatzstoffe hydrophilierte Zahnabdruckmassen.

Bei Zahnabdruckmassen auf der Basis additionsvernetzender oder kondensationsvernetzender Silikone oder auch auf Basis von Polyether-Silikonen und Polyethern besteht das Problem, daß die Zeichnungsschärfe des Abdrucks durch schlechtes Anfließverhalten der Paste infolge unzureichender Hydrophilie nicht befriedigend ist. Diese Problematik ist z.B. in der DE-A-38 38 587, Seite 2, Zeilen 19-23 oder in der EP-A-0 480 238, Seite 2, Zeilen 1-26 dargestellt.

In der Literatur sind zur Lösung dieses Problems verschiedenste Zusätze beschrieben worden, die die Hydrophilie der Abdruckmassen erhöhen. Ein Uberblick über den Stand der Technik findet sich beispielsweise in EP-A-0 480 238, Seite 2, Zeilen 20-38. Als besonders wirksame Zusätze haben sich Polyethersiloxane erwiesen, wie sie beispielsweise in der internationalen Anmeldung WO 87/03001 oder in der EP-B-0 231 420 beschrieben werden.

Sämtliche Zusätze zeigen zwar eine Verbesserung der Hydrophilie der Abdruckmassen; sie weisen jedoch auch eine Reihe von Problemen auf, die im folgenden aufgeführt sind:

- Erhöhte Wasseraufnahme mit der Folge der Dimensionsinstabilität durch Quellung (DE-A-43 06 997, Seite 2, Zeilen 44 und folgende).
- Gasblasenentwicklung bei Silikonabdruckmassen (EP-A-0 480 238, Seite 2, Zeilen 27-38, und DE-A-43 06 997, Seite 2, Zeilen 44-56).
- Schlechte Stabilität (EP-B-0 231 420, Seite 5, Zeilen 13-27).
- Verzögerung der Abbindung (DE-A-43 06 997, Seite 2, Zeilen 53 und folgende).
- Verlust der Hydrophilie nach Desinfektion (DE-A-43 06 997, Seite 2, Zeilen 37 und folgende; sowie Journal of Prosthodontics, Volume 3, Nr. 1, 1994, Seiten 31-34).
- Schlechte Stabilität von Polyethersiloxanen gegenüber Hydrolyse (DE-C-43 20 920, Seite 2, Zeilen 32-36). Damit ist die Langzeitwirkung der Hydrophilierung über den Gebrauchszeitraum einer Abformmasse nicht sicher gewährleistet.

Die oben erwähnten, für die Hydrophilierung von Zahnabdruckmassen besonders geeigneten Polyethersiloxane weisen jedoch einen wesentlichen Nachteil auf. Die mit ihnen hydrophilierten Zahnabdruckmassen sind gegenüber Desinfektionsbädern nicht ausreichend stabil. Die während der Behandlung hergestellten Abdrücke werden in üblicher Weise mindestens zweimal desinfiziert und damit geht ein unerwünschter Verlust an Hydrophilie einher.

Aufgabe der Erfindung ist es, hydrophilierte Zahnabdruckmassen zur Verfügung zu stellen, aus denen Zahnabdrücke mit verbesserter Beständigkeit gegenüber Desinfektionsbädern hergestellt werden können.

Gelöst wird diese Aufgabe durch Zahnabdruckmassen, die 0,1 bis 10 Gew.-%, bezogen auf das Gesamtgewicht der Masse, eines Polyethercarbosilans als Hydrophilierungsmittel enthalten.

Besonders bevorzugt werden als Hydrophilierungsmittel Polyethercarbosilane der folgenden allgemeinen Formel

$$Q - P - (OC_nH_{2n})_x - OT \qquad (I)$$

worin bedeuten:

Q $R_3Si$ -
oder

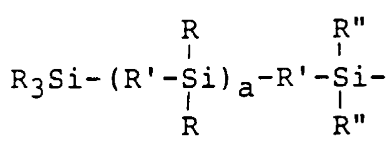

wobei jedes R im Molekül gleich oder verschieden sein kann und einen aliphatischen $C_1$-$C_{18}$-, einen cycloaliphatischen $C_6$-$C_{12}$- oder einen aromatischen $C_6$-$C_{12}$-Kohlenwasserstoffrest, die gegebenenfalls durch Halogenatome substituiert sein können, bedeutet,

R' eine $C_1$-$C_{14}$-Alkylengruppe ist,
R" im Falle von $a \neq 0$ R ist oder im Falle von $a = 0$ R oder $R_3SiR'$ ist, und $a = 0$-2 bedeutet;

P eine $C_2$-$C_{18}$-Alkylengruppe, vorzugsweise eine $C_2$-$C_{14}$-Alkylengruppe oder S-R"', wobei S eine $C_2$-$C_{18}$-Alkylengruppe und R"' eine funktionelle Gruppe aus folgender Liste darstellt:

$-NHC(O)-$, $-NHC(O)(CH_2)_{n-1}-$, $-NHC(O)C(O)-$,
$-NHC(O)(CH_2)_vC(O)-$, $-OC(O)-$, $-OC(O)(CH_2)_{n-1}-$,
$-OC(O)C(O)-$, $-OC(O)(CH_2)_vC(O)-$,
$-OCH_2CH(OH)CH_2OC(O)(CH_2)_{n-1}-$, $-OCH_2CH(OH)CH_2OC(O)(CH_2)_vC(O)$ mit v = 1-12;

T gleich H oder einen $C_1$-$C_4$-Alkylrest oder einen $C_1$-$C_4$-Acylrest;

x eine Zahl von 1 bis 200, und

n eine mittlere Zahl von 1 bis 6, vorzugsweise 1 bis 4.

Der Polyetherteil kann ein Homopolymer sein, kann aber auch ein statistisches-, alternierendes- oder Block-Copolymer sein.

Weiterhin werden Polyethercarbosilane der folgenden allgemeinen Formel als Hydrophilierungsmittel besonders bevorzugt:

$$Q'-(P-(OC_nH_{2n})_x-OT)_2 \qquad (II)$$

worin bedeuten:

Q' $-SiR_2-X-SiR_2-$;

X einen zweiwertigen Kohlenwasserstoffrest, d.h. ein $C_1$-$C_{18}$-Alkylen-, $C_6$-$C_{20}$-Arylen-, $C_5$-$C_{15}$-Cycloalkylen- und $C_5$-$C_{15}$-Polycycloalkylenrest, der gegebenenfalls sauerstoffhaltige Gruppen tragen kann, und wobei

alle weiteren Symbole die gleiche Bedeutung wie in der allgemeinen Formel (I) haben.

Weiterhin werden Polyethercarbosilane der folgenden allgemeinen Formel als Hydrophilierungsmittel besonders bevorzugt:

$$Q-P-(OC_nH_{2n})_x-OP-Q \qquad (III)$$

worin alle Symbole die gleiche Bedeutung haben wie in der allgemeinen Formel (I).

Weiterhin werden als Hydrophilierungsmittel Polyethercarbosilane der folgenden allgemeinen Formel besonders bevorzugt:

$$Q-P'-(OC_nH_{2n})_x-OT \qquad (IV)$$

worin bedeuten:

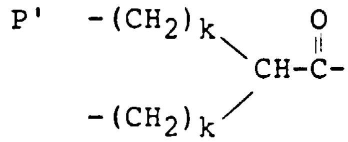

oder

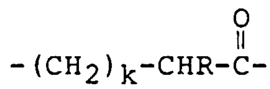

oder

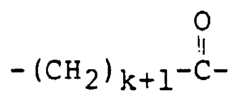

wobei k 1-8 ist; und

alle weiteren Symbole die gleiche Bedeutung wie in der allgemeinen Formel (I) haben.

Schließlich werden als Hydrophilierungsmittel Polyethercarbosilane der folgenden allgemeinen Formel besonders bevorzugt:

$$Q'' [\text{-P-}(OC_nH_{2n})_xOT]_o \qquad (V)$$

worin bedeutet:

Q" ein oligomeres oder polymeres Carbosilan, wobei mindestens ein Siliciumatom, vorzugsweise aber 20 - 70 % der Siliciumatome mit dem Rest -P-$(OC_nH_{2n})_x$OT substituiert sind und damit o ≥ 1 ist; und

alle weiteren Symbole die gleiche Bedeutung wie in der allgemeinen Formel (I) besitzen.

Wie schon erwähnt, weisen aus herkömmlichen Zahnabdruckmassen hergestellte Abdrücke keine genügende Stabilität gegenüber Desinfektionsbädern auf. Durch die Desinfizierung der Abdrücke, die in der Regel mindestens zweimal erfolgt, tritt ein Verlust an Hydrophilie auf, der sich in einem schlechteren Anfließverhalten der wässrigen Gipssuspension beim Ausgießen der Abdrücke bemerkbar macht. Als Maß für das Anfließverhalten kann die Bestimmung des Randwinkels herangezogen werden. Mit dem Verlust an Hydrophilie geht ein zunehmender Randwinkel einher. Der Randwinkel ist der Winkel, den der Rand eines Wassertropfens zur Substratoberfläche bildet (Walter Noll, Chemistry and Technology of Silicone, Academic Press, 1968, insbesondere Seiten 447 bis 452). Das erfindungsgemäße Hydrophilierungsmittel sollte in so ausreichender Menge in der Zahnabdruckmasse vorhanden sein, daß der 10 Sekunden-Randwinkel <55°, vorzugsweise <45°, beträgt.

Die Abnahme der Hydrophilie von beispielsweise mit Polyethersiloxanen hydrophilierten Silikonmassen ist vermutlich darauf zurückzuführen, daß das Hydrophilierungsmittel im Desinfektionsbad herausgelöst wird.

Die Hydrophilierung von Silikonmassen mit Polyethersiloxanen ist in der bereits oben erwähnten internationalen Anmeldung WO 87/03001 und EP-B-0 231 420 beschrieben. Ein geeignetes Polyethersiloxan hat beispielsweise die folgende Formel

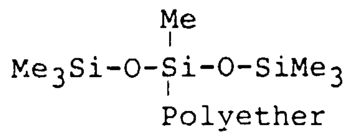

Bei diesen Verbindungen bewirkt die -Si-O-Kette die Veträglichkeit mit den Silikonabdruckmassen, während der Polyetheranteil für die Hydrophilierung und damit für die gute Anfließbarkeit der Masse verantwortlich ist. Eines der erfindungsgemäß geeigneten Polyethercarbosilane weist beispielsweise die folgende Formel auf

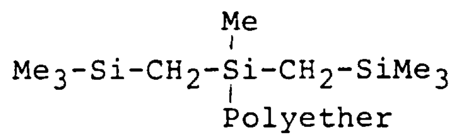

Aufgrund der Struktur der Polyethercarbosilane war zu erwarten, daß ihre Verträglichkeit mit den Silikonmassen aufgrund des Fehlens der Sauerstoffbrücken schlechter ist als dies bei den Polyethersiloxanen der Fall ist. Man mußte daher annehmen, daß sich die erfindungsgemäß einzusetzenden Polyethercarbosilane in den Desinfektionsbädern wesentlich leichter herauslösen lassen als die bekannten Polyethersiloxane, und man mußte daher mit einem noch stärkeren Verlust an Hydrophilie bei der Desinfektion der Abdrücke rechnen. Überraschenderweise ist dies jedoch nicht der Fall. Es hat sich gezeigt, daß sich die aus den mit den erfindungsgemäßen Polyethercarbosilanen hydrophilierten Zahnabdruckmassen hergestellten Abdrücke hinsichtlich der Aufrechterhaltung der Hydrophilie wesentlich günstiger verhalten als Abdrücke, die unter Verwendung von mit Polyethersiloxanen hydrophilierten Silikonmassen hergestellt worden sind.

Die erfindungsgemäßen Zahnabdruckmassen haben darüber hinaus den Vorteil, daß sie stabil sind. In der EP-B-0 231 420 wird auf Seite 5 in den Zeilen 17-27 beschrieben, daß die Stabilität von hydrophilierten Silikonen infolge Feuchtigkeit schlecht sei. Daher muß zur Gewährleistung der Stabilität des hydrophilen Silikons ein wasserab- oder -adsorbierender Füllstoff in die Masse eingebracht werden. Dies ist bei den erfindungsgemäß hydrophilierten Zahnabdruckmassen nicht erforderlich. Sie sind auch ohne den Zusatz derartiger Füllstoffe stabil.

Von Vorteil ist weiterhin, daß die mit den erfindungsgemäßen Zahnabdruckmassen hergestellten Gipsmodelle glatte Oberflächen aufweisen. Der Stand der Technik (z.B. EP-B-0 268 347, Seite 2, Zeilen 7-41 bzw. DE-C-29 26 405, Seite 2, Zeile 46 bis Seite 3, Zeile 10 bzw. DE-A-43 06 997, Seite 2, Zeilen 44-56) beschreibt, daß bei additionsvernetzten Silikonen das Problem der Gasblasenentwicklung besteht, wodurch das Gipsmodell, das durch Ausgießen des gehärteten Abdrucks erhalten wird, eine unerwünschte schwammartige Oberfläche erhält. Um dies zu vermeiden, müssen feinverteiltes Platin oder Palladium als Wasserstoffabsorber zugesetzt werden. Bei den erfindungsgemäßen Zahnabdruckmassen ist dies nicht erforderlich. Ein Gipsmodell, das durch Ausgießen eines Abdruckes aus einem erfindungsgemäßen Zahnabdruckmaterial hergestellt worden ist, weist auch in Abwesenheit von Wasserstoffabsorbern eine glatte Oberfläche auf.

Die Basis der erfindungsgemäßen Zahnabdruckmassen bilden bekannte additionsvernetzende oder kondensationsvernetzende Silikone, Polyether-Silikone oder Polyether. Diese Materialien sind im Stand der Technik ausführlich beschrieben worden, so daß es sich erübrigt, hier näher darauf einzugehen. Additions- oder kondensationsvernetzende Silikone sind beispielsweise in der US-A-3 897 376, in der EP-B-0 231 420 sowie in der dort auf Seite 2 erwähnten US-A-4 035 453, weiterhin in der EP-A-0 480 238 (siehe insbesondere Seite 2, Zeilen 3-26) und in der EP-B-0 268 347 beschrieben. Die Offenbarung dieser Patentschriften soll hier durch Inbezugnahme mitumfaßt sein. Polyether-Silikone sind unter anderem beispielsweise in der DE-A-37 41 575 sowie in der DE-A-38 38 587 beschrieben, deren Offenbarung hier ebenfalls mitumfaßt sein soll. Polyether schließlich sind unter anderem in der DE-B-17 45 810 sowie in der DE-A-43 06 997 beschrieben, deren Offenbarung hier gleichfalls mitumfaßt sein soll.

Wie schon oben erwähnt, enthalten die erfindungsgemäßen Zahnabdruckmassen 0,1 bis 10 Gew.-%, bezogen auf das Gesamtgewicht der Masse, des Polyethercarbosilans als Hydrophilierungsmittel. Vorzugsweise enthalten die Massen 0,5 bis 5 Gew.-% und insbesondere 1 bis 4 Gew.-% des Polyethercarbosilans.

In der oben angegebenen allgemeinen Formel (I) bedeutet R vorzugsweise einen aliphatischen $C_1$-$C_6$-Kohlenwasserstoffrest, insbesondere einen Methyl- oder Ethylrest, einen cycloaliphatischen $C_6$-$C_8$-Kohlenwasserstoffrest oder einen aromatischen $C_6$-$C_8$-Kohlenwasserstoffrest, insbesondere einen Phenylrest.

R' bedeutet vorzugsweise einen $C_1$-$C_6$-Alkylenrest und insbesondere einen Methylen- oder Ethylenrest.

Wenn P ungleich S-R''' ist, dann ist P vorzugsweise eine $C_2$-$C_8$-, insbesondere eine $C_2$-$C_6$-Alkylengruppe. Falls P gleich S-R''' ist, dann ist S vorzugsweise eine $C_2$-$C_8$-, insbesondere eine $C_2$-$C_6$-Alkylengruppe und R''' eine O-C(=O)- oder NH-C(=O)-Gruppe.

T bedeutet vorzugsweise H, einen Methyl- oder Ethylrest oder einen Acetylrest.

a ist vorzugsweise 0, x bedeutet vorzugsweise eine Zahl von 1 bis 50 und n hat vorzugsweise einen Wert von 2 bis 2,5.

Besonders bevorzugte Polyethercarbosilane sind die im folgenden aufgeführten Verbindungen

$$Et_3Si\text{-}(CH_2)_3\text{-}O\text{-}(C_2H_4O)_y\text{-}CH_3$$

$$Et_3Si\text{-}CH_2\text{-}CH_2\text{-}O\text{-}(C_2H_4O)_y\text{-}CH_3$$

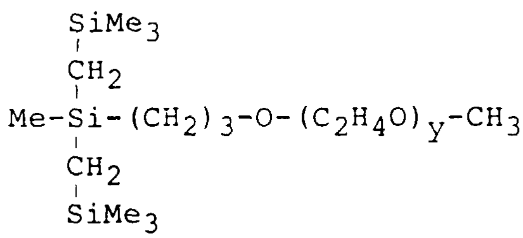

$$(Me_3Si\text{-}CH_2)_3Si\text{-}(CH_2)_3\text{-}O\text{-}(C_2H_4O)_y\text{-}CH_3$$

$$Me_3Si\text{-}CH_2\text{-}SiMe_2\text{-}(CH_2)_3\text{-}O\text{-}(C_2H_4O)_y\text{-}CH_3$$

$$Me_3Si\text{-}(CH_2)_3\text{-}O\text{-}(C_2H_4O)_y\text{-}CH_3$$

$$Me_3Si\text{-}CH_2\text{-}CH_2\text{-}O\text{-}(C_2H_4O)_y\text{-}CH_3$$

$Ph_3Si\text{-}(CH_2)_3\text{-}O\text{-}(C_2H_4O)_y\text{-}CH_3$, Ph = Phenyl

$Ph_3Si\text{-}CH_2\text{-}CH_2\text{-}O\text{-}(C_2H_4O)_y\text{-}CH_3$, Ph = Phenyl

$Cy_3Si\text{-}(CH_2)_3\text{-}O\text{-}(C_2H_4O)_y\text{-}CH_3$, Cy = Cyclohexyl

$Cy_3Si\text{-}CH_2\text{-}CH_2\text{-}O\text{-}(C_2H_4O)_y\text{-}CH_3$, Cy = Cyclohexyl

$$(C_6H_{13})_3Si\text{-}(CH_2)_3\text{-}O\text{-}(C_2H_4O)_y\text{-}CH_3$$

$$(C_6H_{13})_3Si\text{-}CH_2\text{-}CH_2\text{-}O\text{-}(C_2H_4O)_y\text{-}CH_3$$

bei denen y der Beziehung genügt:

$1 \leq y \leq 20$ oder vorzugsweise $1 \leq y \leq 10$.

Diese Verbindungen sind hinsichtlich ihrer Struktur und Herstellung in der DE-C-43 20 920 bzw. in der GB-A-15 20 421 beschrieben. Die EP-A-0 367 381 beschreibt ähnliche Verbindungen mit ionischer Natur. Die Offenbarung

der genannten Patentschriften soll hier durch Inbezugnahme mitumfaßt sein.

Die genannte Patentliteratur beschreibt den Einsatz solcher Polyethercarbosilane mit hydrophilen Gruppen als Tenside im wässrigen Medium oder als schaumkontrollierende Agentien. Einen irgendwie gearteten Hinweis auf den Einsatz als Hydrophilierungsmittel in Dentalmassen findet sich dort nicht.

Die Darstellung der Verbindungen erfolgt nach üblichen Methoden durch Hydrosilylierung entsprechender Polyether mit einer ungesättigten Gruppe der Formel Z - $(OC_nH_{2n})_x$ - OT, wobei Z eine $C_2$-$C_{18}$-Alkenylgruppe, vorzugsweise eine $C_2$-$C_{14}$-Alkenylgruppe oder eine $C_2$-$C_8$-, insbesondere eine $C_2$-$C_6$-Alkenylgruppe ist, mit einem entsprechenden Si-H-haltigen Organosilan der Formel Q-H, wobei bevorzugt ein Katalysator verwendet wird, der aus einem fein verteilten Übergangsmetall oder einer Übergangsmetallverbindung, bevorzugt auf der Basis von Platin, Palladium oder Rhodium, besteht. Es ist bevorzugt, den Katalysator nach der Umsetzung aus dem Carbosilantensid abzutrennen, damit die Lagerstabilität einer Abformmasse, in die das Carbosilantensid als Hydrophilierungsmittel eingebracht wird, nicht gefährdet wird. Die Abtrennung dieses Katalysators kann z.B. durch Adsorption an Kieselgel, Kieselgur oder ähnlichem geschehen.

Für P gleich S-R'''sind besonders bevorzugte Verbindungen

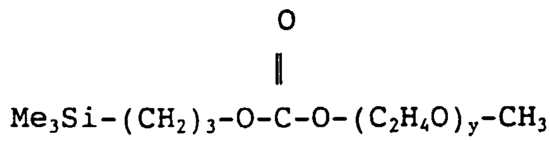

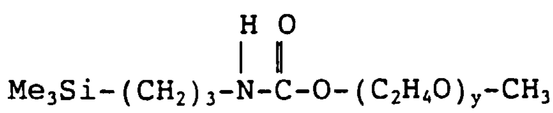

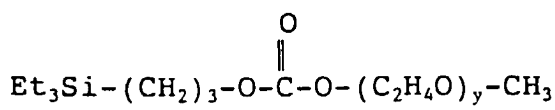

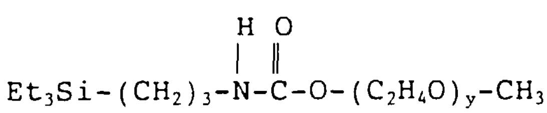

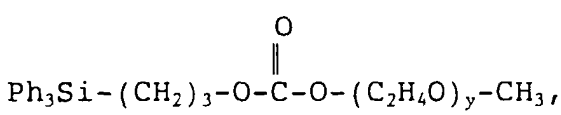
, Ph = Phenyl

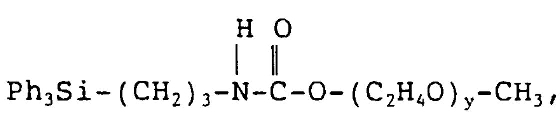
, Ph = Phenyl

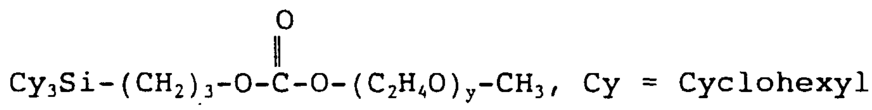

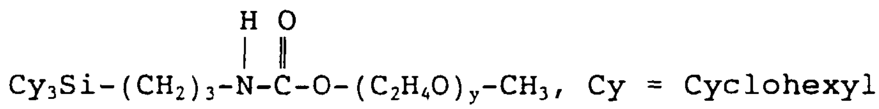

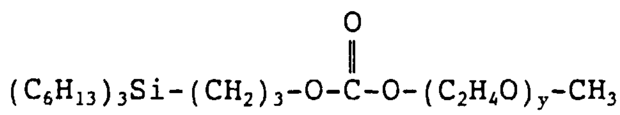

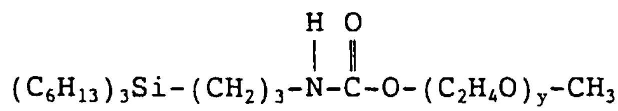

bei denen y der Beziehung genügt:

$1 \leq y \leq 20$ oder vorzugsweise $1 \leq y \leq 10$.

Die Verbindungen der allgemeinen Struktur (I) mit P = S-R''' werden im wesentlichen analog zu literaturbekannten Vorschriften hergestellt. In erster Stufe erfolgt die Herstellung einer ungesättigten Verbindung Z-R'''-$(OC_nH_{2n})_x$-OT, welche durch Hydrosilylierung mit einer Verbindung Q-H in das Produkt mit Struktur (I) analog wie oben beschrieben überführt wird. Im Folgenden sei die Einführung der difunktionellen Gruppen R''' beschrieben.

Für R''' = -NHC(O)- bzw. -OC(O)-: Ausgehend von einem Polyether der Formel H-$(OC_nH_{2n})_x$-OT wird durch Phosgenierung nach Standardverfahren ein reaktiver Chlorameisensäureester der Formel Cl-C(O)-$(OC_nH_{2n})_x$-OT dargestellt (M. Matzner, R. P. Kurkjy, R. J. Cotter, Chem. Rev. 64 (1964) 645). Dieser kann in einfacher Reaktion mit Allylalkohol zu einem Kohlensäure-monoallyl-mono(polyether)ester (U. Petersen in Houben-Weyl Bd. E4, S. 66) der Formel $H_2C$=$CHCH_2$-O-C(O)-$(OC_nH_{2n})_x$-OT oder mit Allylamin zu einem N-Allyl-carbamidsäure-(polyether)-ester (U. Petersen in Houben-Weyl Bd. E4, S. 144) der Formel $H_2C$=$CHCH_2$-NH-C(O)-$(OC_nH_{2n})_x$-OT umgesetzt werden. Geeignet ist prinzipiell jeder andere ungesättigte Alkohol ZOH, bzw. jedes andere ungesättigte Amin der Formel $ZNH_2$, wobei als Produkt die Struktur Z-R'''-$(OC_nH_{2n})_x$-OT erhalten wird. Die erhaltenen Alkenylverbindungen werden mit einem entprechenden Si-H-haltigen Organosilan der Formel Q-H analog zur Herstellung von Struktur (I) in einer Hydrosilylierungsreaktion umgesetzt (I. Ojima in S. Patai, Z. Rappoport "The Chemistry of Organic Silicon Compounds", Wiley 1989), wobei ein Katalysator verwendet wird, der aus fein verteiltem Übergangsmetall oder einer Übergangsmetallverbindung, bevorzugt auf der Basis von Platin, Palladium oder Rhodium, besteht. Es ist bevorzugt, den Katalysator nach der Umsetzung aus dem Carbosilantensid abzutrennen (analog zur Herstellung von Struktur (I) mit P ungleich S-R''').

Für R''' = -NHC(O)C(O)- bzw. -OC(O)C(O)-: Ausgehend von einem Polyether der Formel H-$(OC_nH_{2n})_x$-OT wird durch Umsetzung mit Oxalylchlorid nach Standardverfahren ein reaktiver Chlorglyoxylsäure-ester der Formel Cl-C(O)C(O)-$(OC_nH_{2n})_x$-OT dargestellt (siehe z.B. S. J. Rhoads, R. E. Michel, JACS, Bd. 85 (1963) 585). Dieser kann in einfacher Reaktion mit Allylalkohol zu einem Oxalsäure-monoallyl-mono(polyether)ester der Formel $HC_2$=$CHCH_2$-O-C(O)C(O)-$(OC_nH_{2n})_x$-OT oder mit Allylamin zu einem N-Allyl-mono(polyether)-ester-mono-amid der Formel $H_2C$=$CHCH_2$-NH-C(O)C(O)-$(OC_nH_{2n})_x$-OT umgesetzt werden (A. Jackson, Speciality Chemicals 14/5 (1994) 300). Geeignet ist prinzipiell jeder andere ungesättigte Alkohol ZOH, bzw. jedes andere ungesättigte Amin der Formel $ZNH_2$, wobei als Produkt die Struktur Z-R'''-$(OC_nH_{2n})_x$-OT erhalten wird. Die erhaltenen Alkenylverbindungen werden mit einem entprechenden Si-H-haltigen Organosilan der Formel Q-H in einer Hydrosilylierungsreaktion zu den entsprechenden Carbosilantensiden umgesetzt. Hierbei gilt für Katalyse und Aufarbeitung das oben gesagte.

Für R''' = -NHC(O)$(CH_2)_{n-1}$-, -OC(O)$(CH_2)_{n-1}$- bzw. -$OCH_2CH(OH)CH_2OC(O)(CH_2)_{n-1}$-: Ausgehend von einem Polyether der Formel H-$(OC_nH_{2n})_{x+1}$-OT wird durch Oxidation mit Salpetersäure ein säurefunktionalisierter Polyether der Formel HOC(O)$(CH_2)_{n-1}$-$(OC_nH_{2n})_x$-OT erzeugt (H. Henecka in Houben-Weyl Bd. 8, S. 409). Die erzeugte

Säure wird beispielsweise mittels Thionylchlorid in das Säurechlorid der Formel $ClC(O)(CH_2)_{n-1}-(OC_nH_{2n})_x-OT$ (H. Henecka, Houben-Weyl Bd. 8 S. 463, R. Sustmann, H.-G. Korth, Houben-Weyl Bd. E5/1 S. 593) überführt und dieses mittels Allylalkohol in den Allylester der Formel $H_2C=CHCH_2-O-C(O)(CH_2)_{n-1}-(OC_nH_{2n})_x-OT$ (H. Henecka in Houben-Weyl Bd. 8, S. 543) oder mit Allylamin in das N-Allylamid der Formel $H_2C=CHCH_2-NH-C(O)(CH_2)_{n-1}-(OC_nH_{2n})_x-OT$ (H. Henecka, P. Kurtz in Houben-Weyl Bd. 8, S. 655) überführt. Geeignet ist prinzipiell jeder andere ungesättigte Alkohol ZOH, bzw. jedes andere ungesättigte Amin der Formel $ZNH_2$, wobei als Produkt die Struktur $Z-R'''-(OC_nH_{2n})_x-OT$ erhalten wird.

Die ringöffnende Umsetzung des säurefunktionalisierten Polyethers der Formel $HOC(O)(CH_2)_{n-1}-(OC_nH_{2n})_x-OT$ mit Allylglycidyl-ether führt zu Verbindungen der Formel $H_2C=CHCH_2-OCH_2CH(OH)CH_2-OC(O)(CH_2)_{n-1}-(OC_nH_{2n})_x-OT$ (H. Henecka in Houben-Weyl Bd. 8, S. 531).

Die erhaltenen Alkenylverbindungen werden mit einem entprechenden Si-H-haltigen Organosilan der Formel Q-H in einer Hydrosilylierungsreaktion zu den entsprechenden Carbosilantensiden umgesetzt. Hierbei gilt für Katalyse und Aufarbeitung das oben gesagte.

Für R''' = $-OC(O)(CH_2)_vC(O)-$, $-NHC(O)(CH_2)_vC(O)-$ bzw. $-OCH_2CH(OH)CH_2OC(O)(CH_2)_vC(O)-$: Ausgehend von einem Polyether der Formel $H-(OC_nH_{2n})_x-OT$ wird durch äquimolare Umsetzung mit einem difunktionellen Säurechlorid der Formel $ClC(O)(CH_2)_vC(O)Cl$ nach Standardverfahren eine reaktive Verbindung der Formel $ClC(O)(CH_2)_vC(O)-(OC_nH_{2n})_x-OT$ erhalten. Durch anschließende Veresterung mit Allylalkohol (H. Henecka in Houben-Weyl Bd. 8, S. 543) entstehen Ester der Formel $H_2C=CHCH_2-O-C(O)(CH_2)_vC(O)-(OC_nH_{2n})_x-OT$; die Umsetzung mit Allylamin führt zu N-Allylamiden der Formel $H_2C=CHCH_2NH-C(O)(CH_2)_vC(O)-(OC_nH_{2n})_x-OT$ (H. Henecka, P. Kurtz in Houben-Weyl Bd. 8, S. 655). Geeignet ist prinzipiell jeder andere ungesättigte Alkohol ZOH, bzw. jedes andere ungesättigte Amin der Formel $ZNH_2$, wobei als Produkt die Struktur $Z-R'''-(OC_nH_{2n})_x-OT$ erhalten wird.

Die Umsetzung der freien Säure (erhältlich durch Hydrolyse des Säurechlorids $ClC(O)(CH_2)_vC(O)-(OC_nH_{2n})_x-OT$) mit Allylglycidylether führt zu Verbindungen der Formel $H_2C=CHCH_2-O-CH_2CH(OH)CH_2OC(O)(CH_2)_vC(O)-(OC_nH_{2n})_x-OT$ (H. Henecka in Houben-Weyl Bd. 8, S. 531). Die erhaltenen Alkenylverbindungen werden mit einem entprechenden Si-H-haltigen Organosilan der Formel Q-H in einer Hydrosilylierungsreaktion zu den entsprechenden Carbosilantensiden umgesetzt. Hierbei gilt für Katalyse und Aufarbeitung das oben gesagte.

In der oben angegebenen allgemeinen Strukturformel (II) bedeutet R vorzugsweise einen aliphatischen $C_1$-$C_6$-Kohlenwasserstoffrest, insbesondere einen Methyl- oder Ethylrest, einen cycloaliphatischen $C_6$-$C_8$-Kohlenwasserstoffrest oder einen aromatischen $C_6$-$C_8$-Kohlenwasserstoffrest, insbesondere einen Phenylrest.

X bedeutet vorzugsweise eine $C_2$-$C_{10}$-, insbesondere eine $C_4$-$C_8$-Alkylengruppe, eine $C_6$-$C_{15}$-Arylen, insbesondere p-Phenylen, eine cykloaliphatische $C_5$-$C_{10}$-, insbesondere $C_5$-$C_7$-Cycloalkylengruppe oder eine polycykloaliphatische $C_8$-$C_{12}$-Alkylengruppe-, ganz besonders Tricyclo[5.2.1.$0^{2,6}$]dec-3(4),8(9)-ylen.

P ist vorzugsweise eine $C_2$-$C_8$-, insbesondere eine $C_2$-$C_6$-Alkylengruppe.

T bedeutet vorzugsweise H, einen Methyl- oder Ethylrest oder einen Acetylrest.

x bedeutet vorzugsweise eine Zahl von 1 bis 50 und n hat vorzugsweise einen Wert von 2 bis 2,5.

Besonders bevorzugte Polyethercarbosilane sind die im folgenden aufgeführten Verbindungen

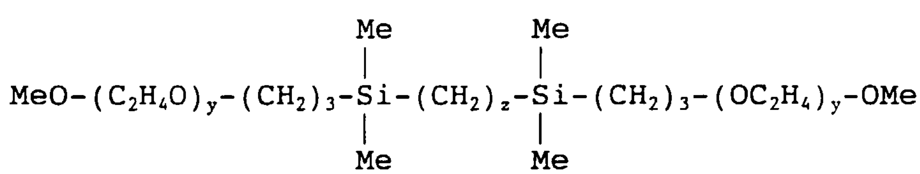

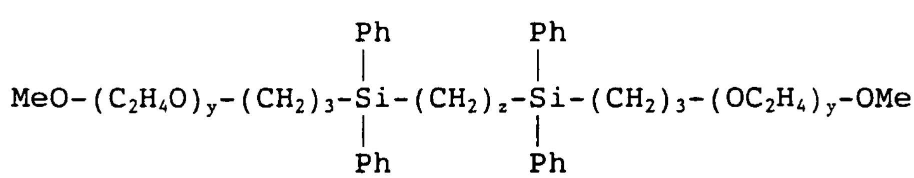

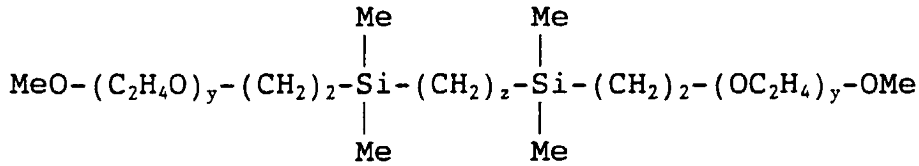

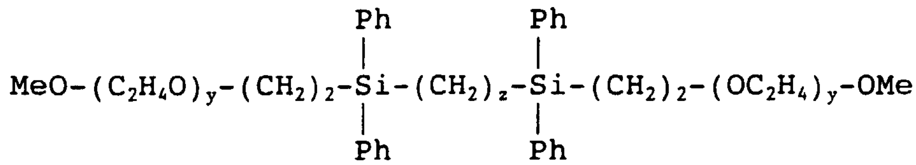

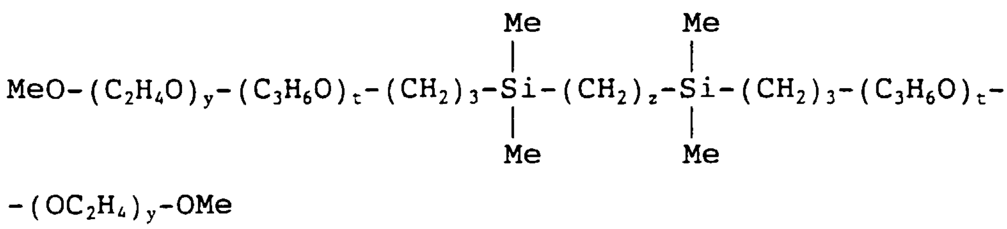

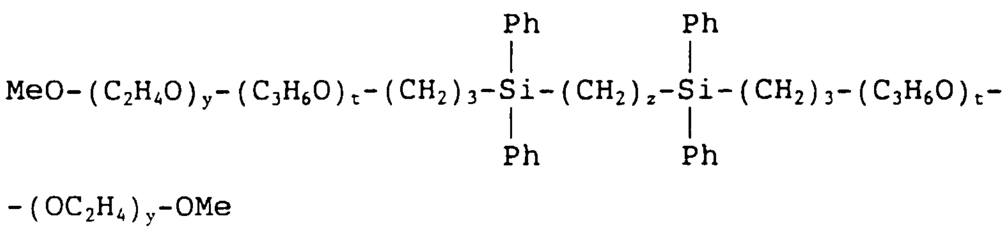

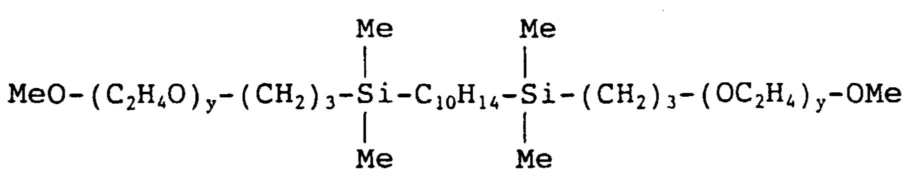

$C_{10}H_{14}$ = Tricyclo[5.2.1.$0^{2,6}$]dec-3(4),8(9)-ylen

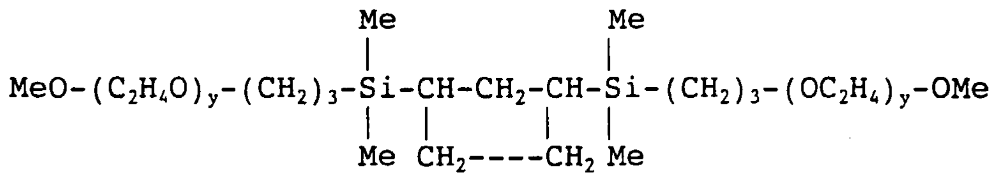

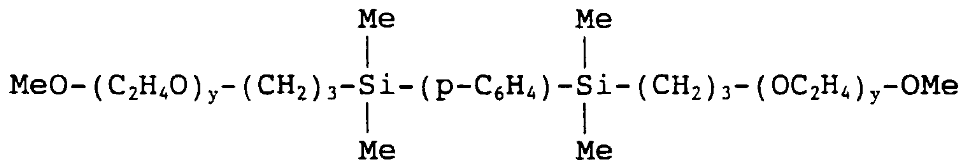

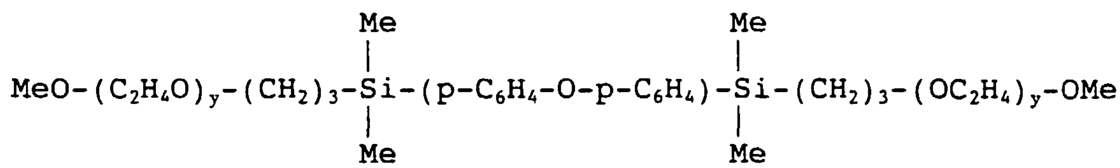

bei denen y der Beziehung genügt:

$1 \leq y \leq 20$ oder vorzugsweise $1 \leq y \leq 10$,

und z der Beziehung genügt:

$2 \leq z \leq 8$ oder vorzugsweise $2 \leq z \leq 6$;

und t der Beziehung genügt:

$0{,}1 \cdot y \leq t \leq y$ oder vorzugsweise $0{,}15 \cdot y \leq t \leq 0{,}55 \cdot y$.

Die Verbindungen der allgemeinen Struktur (II) werden in der Art dargestellt, daß vorerst das bis-organosilylmodifizierte Mittelstück $-SiR_2XSiR_2-$ beispielsweise mittels Kupplungsreaktion aus einem Chlor-funktionalisierten Silan $HSiR_2Cl$ und einer Di-Grignard-Verbindung ClMgXMgCl (K. Nützel in Houben-Weyl, Bd. 13/2a S. 101, S. Pawlenko in Houben-Weyl Bd. 13/5 S. 85) oder durch Bis-Hydrosilylierung eines Alkadiens mit einem Organo-H-Silan z. B. $R_2SiHCl$ erhalten wird (I. Ojima in S. Patai, Z. Rappoport "The Chemistry of Organic Silicon Compounds", Wiley 1989 oder S. Pawlenko in Houben-Weyl Bd 13/5 S. 85). Dieser Rest Q' = $-SiR_2XSiR_2-$ ist entweder bereits Si-H-terminiert, oder wird für die weitere Umsetzung nachträglich durch Hydrierung von Si-Cl-Gruppen mit $LiAlH_4$ zu Verbindungen der Formel $H-SiR_2XSiR_2-H$ funktionalisiert. Diese Verbindung wird mit ungesättigten Polyetherresten der Formel $Z-(OC_nH_{2n})_x-OT$, wobei Z wie oben definiert ist, in einer Hydrosilylierungsreaktion (analog zur Herstellung und Aufarbeitung von Struktur (I)) verknüpft.

Falls P gleich S-R''' ist, erfolgt die Einführung dieser Gruppe analog zu den bei Struktur (I) beschriebenen Verfahren.

In der oben angegebenen allgemeinen Strukturformel (III) bedeutet R vorzugsweise einen aliphatischen $C_1$-$C_6$-Kohlenwasserstoffrest, insbesondere einen Methyl- oder Ethylrest, einen cycloaliphatischen $C_6$-$C_8$-Kohlenwasserstoffrest oder einen aromatischen $C_6$-$C_8$-Kohlenwasserstoffrest, insbesondere einen Phenylrest.

R' bedeutet vorzugsweise einen $C_1$-$C_6$-Alkylenrest und insbesondere einen Methylen- oder Ethylenrest.

P ist vorzugsweise eine $C_2$-$C_8$-, insbesondere eine $C_2$-$C_6$-Alkylengruppe.

a ist vorzugsweise 0, x bedeutet vorzugsweise eine Zahl von 1 bis 50 und n hat vorzugsweise einen Wert von 2 bis 2,5.

Besonders bevorzugte Polyethercarbosilane sind die im folgenden aufgeführten Verbindungen

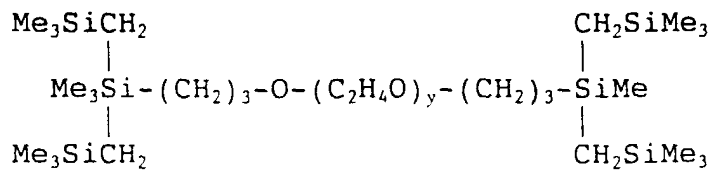

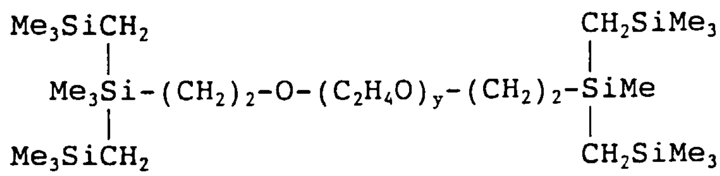

$(Cy)_2MeSi\text{-}(CH_2)_3\text{-}O\text{-}(C_2H_4O)_y\text{-}(CH_2)_3\text{-}SiMe(Cy)_2$, Cy = Cyclohexyl

$(Cy)_2MeSi\text{-}(CH_2)_2\text{-}O\text{-}(C_2H_4O)_y\text{-}(CH_2)_2\text{-}SiMe(Cy)_2$, Cy = Cyclohexyl

bei denen y der Beziehung genügt:

$1 \leq y \leq 30$ oder vorzugsweise $2 \leq y \leq 20$.

Die Verbindungen der allgemeinen Struktur (III) werden ausgehend von ungesättigten Polyethern der Formel $Z\text{-}(OC_nH_{2n})_x\text{-}O\text{-}Z$, wobei Z wie oben definiert ist, in einer Hydrosilylierungsreaktion mit Si-H-haltigen Organosilanen der Form Q-H (analog zur Herstellung von Struktur (I)) verknüpft. Die Herstellung der linearen ungesättigten Verbindungen $Z\text{-}(OC_nH_{2n})_x\text{-}O\text{-}Z$ wird beispielsweise in DE-A 37 41 575 beschrieben.

Falls P gleich S-R''' ist, erfolgt die Einführung dieser Gruppe analog zu den bei Struktur (I) beschriebenen Verfahren.

In der oben angegebenen allgemeinen Strukturformel (IV) bedeutet Q vorzugsweise $R_3Si$-. R bedeutet vorzugsweise einen aliphatischen $C_1$-$C_6$-Kohlenwasserstoffrest, insbesondere einen Methyl- oder Ethylrest, einen cycloaliphatischen $C_6$-$C_8$-Kohlenwasserstoffrest oder einen aromatischen $C_6$-$C_8$-Kohlenwasserstoffrest, insbesondere einen Phenylrest.

P' ist vorzugsweise entweder eine 2,2-Bis-alkylen-acetyl-Gruppe ($-[(CH_2)_k]_2CHC(O)-$) mit $C_1$-$C_5$- (k = 1-5), insbesondere $C_1$-$C_3$-Alkylengruppen (k = 1-3), eine 2-Alkylen-2-alkyl-acetyl-Gruppe ($-[(CH_2)_k]CHRC(O)-$) mit einer $C_1$-$C_5$- (k= 1-5), insbesondere einer $C_1$-$C_3$-Alkylen (k = 1-3) und einer Alkylgruppe R = $C_2$-$C_{14}$ oder eine 2-Alkylen-acetylgruppe ($-[(CH_2)_k]CH_2C(O)-$) mit einer $C_1$-$C_5$- (k = 1-5), insbesondere einer $C_1$-$C_3$-Alkylengruppe (k = 1-3).

T bedeutet vorzugsweise H, einen Methyl- oder Ethylrest oder einen Acetylrest.

x bedeutet vorzugsweise eine Zahl von 1 bis 50 und n hat vorzugsweise einen Wert von 2 bis 2,5.

Besonders bevorzugte Polyethercarbosilane sind die im folgenden aufgeführten Verbindungen

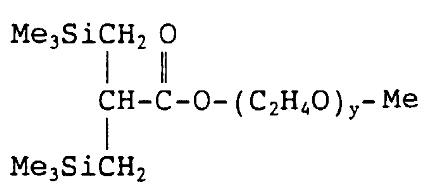

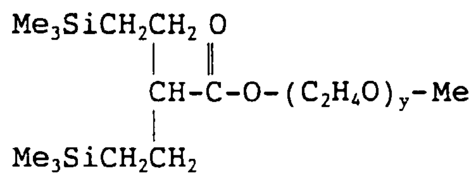

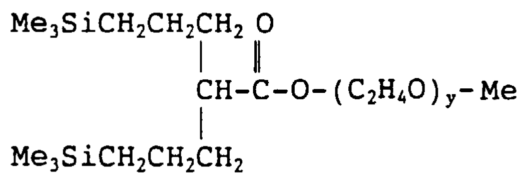

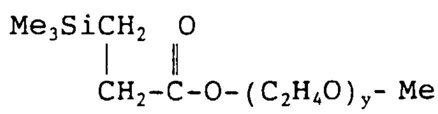

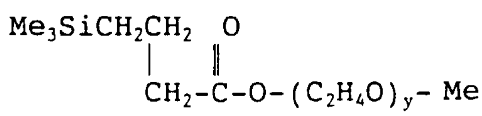

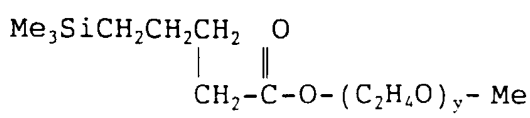

bei denen y der Beziehung genügt:

$1 \leq y \leq 20$ oder vorzugsweise $1 \leq y \leq 10$.

Die Verbindungen der allgemeinen Struktur (IV) werden ausgehend von Malonsäuredialkylester durch Deprotonierung dieses Esters mit starken Basen und Alkylierung des entstandenen Carbanions mit Verbindungen der Formel $Q\text{-}(CH_2)_kCl$ bzw. R-Cl hergestellt. Im Falle der zweifach alkylierten Verbindungen mit $P' = (\text{-}(CH_2)_k)_2CHC(O)\text{-}$ bzw. $\text{-}(CH_2)_kCHRC(O)$-wird diese Prozedur zweimal durchgeführt. Die entstandenen alkylierten Malonester-Derivate werden alkalisch verseift und thermisch decarboxyliert oder im sauren Milieu simultan verseift und decarboxyliert (S. Pawlenko in Houben-Weyl Bd. 13/5, 1980, S. 21, 75; L.H. Sommer, J.R. Gold, M. Goldberg, M.S. Marans, 71 (1949) 1507, H. Henecka in Houben-Weyl Bd. 8 S. 600). Die entstandenen freien Monocarbonsäuren der Form QP'-OH werden mittels Schleppveresterung mit einem Polyether der Formel $H\text{-}(OC_nH_{2n})_xOT$ verestert (H. Henecka in Houben-Weyl Bd. 8 S. 522).

Ein alternativer Weg ist die Darstellung von alkylierten Malonestern der Formel $Z_{2-j}R_jC(COOR)_2$ mit j = 0 oder 1 durch Alkylierung von Malonsäuredialkylester mit RCl und ZCl (z.B. Allylchlorid) unter Einfluß einer starken Base.

Nach Verseifung und Decarboxylierung kann diese substituierte Carbonsäure mit dem oben erwähnten Polyether der Formel $H(OC_nH_{2n})_xOT$ verestert werden (H. Henecka in Houben-Weyl Bd. 8 S. 522). Das entstandene Produkt wird nun mit einem Si-H-haltigen Organosilan Q-H durch Hydrosilyierung (analog zur Herstellung und Aufarbeitung von Struktur (I)) zu Carbosilantensiden der Struktur (IV) verknüpft.

In der oben angegebenen allgemeinen Strukturformel (V) bedeutet Q" ein oligomeres oder polymeres Carbosilan, bei dem vorzugsweise 20 - 70 % der Siliziumatome mit einem Rest $-[P-(OC_nH_{2n})_x-OT]$ substituiert sind. Ansonsten tragen die Si-Atome vorzugsweise Methyl-, Ethyl- oder Phenylreste. Die Carbosilankette besteht neben den Silicium-Atomen vorzugsweise aus L-Gruppen, wobei L vorzugsweise ein Alkylenrest $C_1$-$C_3$ oder ein p-Phenylenrest ist. Die Kette trägt als Endgruppen Methyl-, Ethyl-, Propyl- oder Phenylgruppen.

P ist vorzugsweise eine $C_2$-$C_8$-, insbesondere eine $C_2$-$C_6$-Alkylengruppe.

T bedeutet vorzugsweise H, einen Methyl- oder Ethylrest oder einen Acetylrest.

x bedeutet eine Zahl von 1 bis 200, vorzugsweise eine Zahl von 1 bis 50, und n ist eine mittlere Zahl von 1 bis 6, vorzugsweise ein Wert von 2 bis 2,5.

Besonders bevorzugte Polyethercarbosilane sind die im folgenden aufgeführten Verbindungen

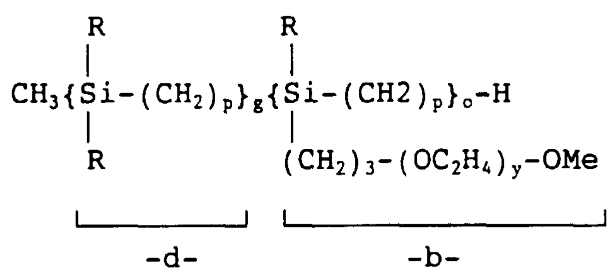

bei denen y der Beziehung genügt:

$1 \leq y \leq 20$ oder vorzugsweise $1 \leq y \leq 10$; und p gleich 2 oder 3 sein kann;

o sei vorzugsweise 3-1000 und g insbesondere der Beziehung genügt:

$4 \cdot o \geq g \geq 0{,}42 \cdot o$.

R ist in diesem Fall vorzugsweise Methyl, Ethyl oder Phenyl.

Die Carbosilankette kann aus alternierenden, statistischen oder blockcopolymeren Einheiten bestehen.

Ein Syntheseweg zu derartigen Carbosilantensiden ist die Umsetzung von Organohydrogensilanen mit 2 SiH-Gruppen mit einem Organosilan mit zwei ungesättigten Gruppen in einer Hydrosilylierungsreaktion, wobei mindestens eines der genannten Organosilane eine Gruppe tragen muß, die in eine Si-H-Einheit überführt werden kann - beispielsweise eine Halogengruppe, die durch Hydrierung beispielsweise mit Lithiumalanat reduziert werden kann - und die die Hydrosilylierungsreaktion nicht negativ beeinflußt. Beispiele für die difunktionellen Organohydrogensilane sind Diphenylsilan oder Phenylmethylsilan, Methylbromsilan oder Phenylchlorsilan. Beispiele für die ungesättigten Silane sind Divinyl-dimethylsilan, Diallyl-dimethylsilan, Divinyldiphenylsilan, Diallyl-diphenylsilan, Divinyl-methylchlorsilan oder Divinyl-methyl-bromsilan, Diallyl-methylchlorsilan oder Diallyl-methyl-bromsilan. Das Hydrosilylierungsprodukt ist ein polymerer Stoff, dessen Molekulargewicht durch Zugabe von monofunktionellen ungesättigten Silanen - z. B. Vinyltrimethylsilan - oder Si-H-Verbindungen - z. B. Triphenylsilan oder Triethylsilan-eingestellt werden kann (S. Pawlenko in Houben-Weyl Bd. 13/5 S. 328).

Nach anschließender Hydrierung der Si-Halogen-Gruppe zur Si-H-Gruppe mit $LiAlH_4$ wird mit einem ungesättigten Polyether der Formel $Z-(OC_nH_{2n})_x-OT$, wobei Z wie oben definiert ist, unter Platinkatalyse (analog zur Herstellung von Struktur (I)) zum Carbosilantensid hydrosilyliert.

Eine weitere Möglichkeit der Darstellung eines Carbosilantensides der allgemeinen Struktur (V) besteht darin, nach bekannten Verfahren Silacyclobutane der Formel $(CH_2)_3SiHR$ herzustellen (N.S. Nametkine, V.M. Vdovine, J. Polym. Sci C (1963) 1043, R. Damrauer, Organometal. Chem. Rev. A, 8 (1972) 67).

Diese Silacyclobutane können mit ungesättigten Polyethern der Formel $Z-(OC_nH_{2n})_x-OT$, wobei Z wie oben definiert ist, unter Platinkatalyse, beispielsweise nach in obiger Literatur beschriebenen Verfahren, hydrosilyliert und danach - gegebenenfalls unter Zusatz von Silacyclobutanen ohne Polyetherresten - zu Carbosilantensiden der allgemeinen Formel (V) ringöffnend polymerisiert werden.

Die bei dieser Hydrosilylierung entstandenen Silacyclobutane der Formel $(CH_2)_3SiR-P-(OC_nH_{2n})_x-OT$ können beispielsweise thermisch polymerisiert werden, z.B. analog zu den in US 3 046 291 beschriebenen Verfahren. Die Polymerisation kann auch in Gegenwart eines Ubergangs-Metallkatalysators, bevorzugt auf Platinbasis (siehe z.B. D. R. Weyenberg, L.E. Nelson, Journal of Organic Chemistry, 30 (1965) 2618 oder US 3 445 495) erfolgen. Denkbar ist

auch eine Beschleunigung der Polymerisation durch anionische Verbindungen, wie KOH oder Lithiumalkyle (z.B. C. X. Liao, W.P. Weber, Polymer Bulletin 28 (1992) 281. Die Polymerisation kann mit oder ohne Lösungsmittel erfolgen.

Ein anderer Weg besteht darin, die SiH-tragenden Silacyclobutane nach bekannten Verfahren unter Ringöffnung zu polymerisieren und anschließend die SiH-haltigen Polycarbosilane mit Polyethern der Formel Z-$(OC_nH_{2n})_x$-OT, wobei Z wie oben definiert ist, zu hydrosilylieren (C.X. Liao, W.P. Weber, Macromol. 26(4) (1993) 563).

Prinzipiell können SiH-haltige Poly- oder Oligocarbosilane auch durch Umsetzung von Digrignard-Verbindungen mit $Cl_2SiMeH$ unter Verwendung von $ClSiMe_3$ oder $ClSiMe_2H$ als Kettenabbrecher hergestellt werden. Anschließend erfolgt wiederum analog zu oben die Hydrosilylierung der Produkte mit den ungesättigten Polyethern.

Bei allen geschilderten Herstellungsverfahren durch Hydrosilylierung wird vorzugsweise der Platinkatalysator nach der Herstellung durch bereits geschilderte Verfahren entfernt.

Die nachfolgenden Beispiele dienen der Erläuterung der Erfindung. Die Erfindung ist nicht auf die Beispiele beschränkt.

## Beispiele

Die Messung des Randwinkels erfolgt mit einem Kontaktwinkelmeßsystem G1/G40 (Krüss). Das Randwinkelmeßgerät G1 ermöglicht die präzise Abbildung von Tropfenprofilen auf Festkörperoberflächen. Das G40-Meßsystem umfaßt einen Videotubus mit Strahlenteiler, so daß die gleichzeitige Beobachtung eines Tropfens durch das Goniometerokular (Tropfengröße) und die Videokamera (digitale Bildauswertung) ermöglicht wird.

Die Messung erfolgt am liegenden Tropfen bei 23°C und 50 % relativer Luftfeuchtigkeit. 30 Minuten nach Anmischbeginn der Massen wird ein stets gleich großer Tropfen einer bei 23°C gesättigten Calciumsulfatdihydratlösung auf das zwischen Glasplatten zu glatter Oberfläche ausgehärtete Elastomer aufgetragen und sofort mit der Messung begonnen. Der 10-Sekunden-Wert wird zur Auswertung herangezogen.

In den Beispielen ist der jeweilige HLB-Wert der Hydrophilierungsmittel angegeben. Der HLB-Wert ist ein Maß für die Wasser- und Fettfreundlichkeit von Verbindungen (s. Ullmann's Encyclopedia of Industrial Chemistry, Vol. A25, Seite 784 ff. (1994)). Der HLB-Wert der erfindungsgemäßen Hydrophilierungsmittel sollte in der Regel zwischen etwa 5 und 15 liegen.

## Herstellungsbeispiel 1

Zu einer Lösung von 4,7 g Triethylsilan in 28 ml Toluol werden 9,0 g eines Monomethyl-monoallyl-terminierten Polyethylenglykols mit einem mittleren Molekulargewicht von 450 gegeben. Es wird ein Platinkatalysator hinzugefügt und die Reaktionsmischung so lange bei 100°C gerührt, bis laut $^1$H-NMR-Spektrum des Rohproduktes keine Allylgruppen mehr vorhanden sind. Nach Filtration über Kieselgel und Entfernen der flüchtigen Bestandteile im Vakuum werden 4,0 g einer Verbindung 1 erhalten, deren NMR-Spektrum mit der folgenden Strukturformel konsistent ist (n = ca. 7-10):

$$Et_3Si\text{-}(CH_2)_3\text{-}(OC_2H_4)_n\text{-}OCH_3$$

Der HLB-Wert beträgt ca. 14.

## Anwendungsbeispiel 1

28,2 Teile eines Vinyl-endgestoppten Polydimethylsiloxans mit einer Viskosität von 2000 mPa.s bei 23°C, 2,5 Teile eines SiH-Gruppen-haltigen Polydimethylsiloxans mit einer Viskosität von 60 mPa.s bei 23°C, 9,7 Teile eines Polydimethylsiloxans mit einer Viskosität von 50 mPa.s bei 23°C, 8,1 Teile einer silanisierten pyrogenen Kieselsäure, 49,1 Teile Quarzfeinstmehl, 0,3 Teile anorganisches Farbpigment und 2,3 Teile des Hydrophilierungsmittels aus dem Herstellungsbeispiel 1 werden in einem Kneter durch Mischen zu einer homogenen Basispaste vereinigt.

Die Katalysatorpaste wird durch Vermischen von 20,6 Teilen eines Vinyl-endgestoppten Polydimethylsiloxans mit einer Viskosität von 2000 mPa.s bei 23°C, 7,5 Teilen einer silanisierten pyrogenen Kieselsäure, 53,1 Teilen Quarzfeinstmehl, 13,3 Teilen eines Polydimethylsiloxans mit einer Viskosität von 50 mPa.s bei 23°C, 3,4 Teilen einer Lösung von 1,3 Teilen eines Komplexes aus Platin und Divinyltetramethyldisiloxan in einem Polydimethylsiloxan mit einer Viskosität von 50 mPa.s bei 23°C und 2,3 Teilen des Hydrophilierungsmittels aus dem Herstellungsbeispiel 1 hergestellt.

### Messung des Benetzungs-Randwinkels

50 g Basispaste und 10 g Katalysatorpaste werden vollständig vermischt. Nach einigen Minuten erhält man

eine gummielastische Masse. 30 Minuten nach Herstellung wird der Randwinkel zu 42° bestimmt. Anschließend werden die Prüfkörper im handelsüblichen Desinfektions-Tauchbad (Impresept, ESPE) 10 Minuten desinfiziert. Die Prüfkörper werden entnommen, unter fließendem kalten Wasser kurz gespült und 2 Stunden an Luft von 23°C und 50 % relativer Feuchte trocknen gelassen. Danach wird wieder der Randwinkel zu 54° bestimmt.

Zum Vergleich werden entsprechende Massen mit zwei handelsüblichen Polyethersiloxan-Tensiden "Silwet" L-77 und "Tegopren" 5878 hydrophiliert. Unter gleichen Bedingungen wird der Randwinkel vor und nach Desinfektion bestimmt. Das Ergebnis ist in der folgenden Tabelle zusammengefaßt.

Weiterhin wurde eine im Handel erhältliche hydrophilierte Silikon-Zahnabdruckmasse "Reprosil" (eine Masse gemäß EP-B-0 231 420) zu Vergleichszwecken untersucht. Das Ergebnis ist gleichfalls in der nachfolgenden Tabelle angegeben.

Bei einer weiteren Desinfektionsbehandlung, wie sie in der Praxis regelmäßig erfolgt, werden die Unterschiede in den relativen Randwinkelerhöhungen zwischen den erfindungsgemäßen Massen und den bekannten Massen noch größer.

Anwendungstechnische Prüfung

50 g Basispaste und 10 g Katalysatorpaste werden innerhalb von 30 Sekunden vollständig miteinander vermischt. Mit der angemischten Masse wird ein Dimensionsprüfkörper nach ADA 19 abgeformt. Nach einigen Minuten erhält man eine gummielastische Abformung des Prüfkörpers. Diese Abformung wird 30 Minuten nach Anmischbeginn mit einer wässrigen Gipsaufschlämmung (100 g Geostone /23 g Wasser) ausgegossen. Das nach Gipsabbindung erhaltene Modell weist eine einwandfreie Oberfläche auf.

Dimensionsstabilität

Nach ADA 19 wird die Dimensionsstabilität zu -0,1 % bestimmt.

Messung der Lagerstabilität

Basispaste und Katalysatorpaste werden voneinander getrennt über einen Zeitraum von 14 Tagen bei 70°C gelagert. Nach diesem Zeitraum werden beide Pasten auf 23°C abgekühlt. 50 g Basispaste und 10 g Katalysatorpaste aus diesem Alterungstest werden vollständig miteinander vermischt. Der Verlauf der Abbindung wird an einem Cycloviskographen bestimmt. Der Abbindeverlauf unterscheidet sich nicht von jenem der frisch hergestellten, nicht gealterten Pasten.

TABELLE

| Beispiel | Hydrophilierungsmittel | RW vor Desinf. | RW nach Desinf. | relative RW-Erhöhung |
|---|---|---|---|---|
| 2 | gemäß Herstellungsbeispiel | 42° | 54° | 28 % |
| Vergl.1 | Silwet L-77 | 45° | 72° | 60 % |
| Vergl.2 | Tegopren 5878 | 47° | 74° | 57 % |
| Reprosil | gemäß EP-B-0 231 420 | 45° | 65° | 44 % |

**Herstellungsbeispiel 2**

Analog zur Herstellvorschrift in EP-A-0 367 381 (Beispiel 1, Seite 4) wurde Bis(trimethylsilylmethyl)methylsilan hergestellt. Dieses wurde analog Beispiel 1 in GB-A-1 520 421 mit einem Methylallyl-terminierten Polyethylenglykol mit einem mittleren Molekulargewicht von 350 in einer Hydrosilylierungsreaktion umgesetzt. Nach Aufarbeitung analog Herstellungsbeispiel 1 wurde eine Verbindung 2 erhalten, deren NMR-Spektrum mit der folgenden Strukturformel konsistent ist (n = ca. 5-8):

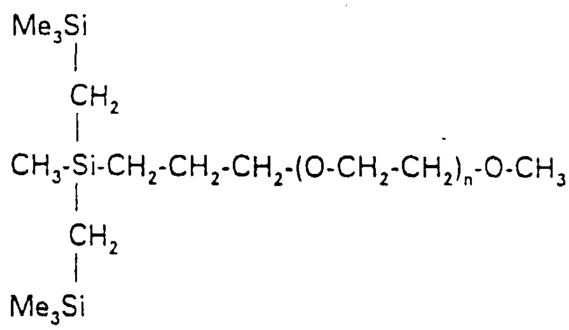

Der HLB-Wert des Materials beträgt ca. 11.

Bei der Herstellung von Bis(trimethylsilylmethyl)methylsilan fällt als Nebenprodukt ein Disilan mit folgender Struktur an:

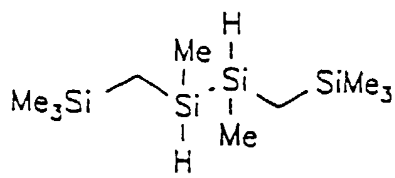

Die Substanz wird bevorzugt bei der Aufarbeitung z.B. durch Destillation abgetrennt. Die Substanz kann jedoch, wenn sie in nur geringen Anteilen anfällt, auch im Herstellungsprodukt belassen werden und ergibt bei der Hydrosilylierung folgende Struktur:

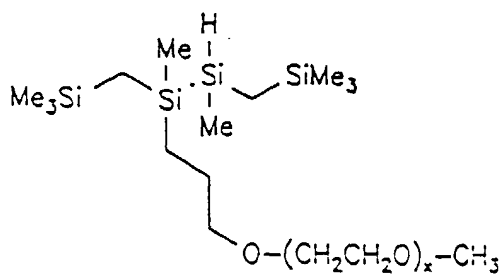

x = 5 - 8

Hierbei handelt es sich ebenfalls um ein Carbosilantensid mit hydrophilierender Wirkung. Die Möglichkeit der Herstellung von Carbosilantensiden mit Disilanstruktur ist nicht auf dieses Beispiel beschränkt.

<u>**Anwendungsbeispiel 2**</u>

Analog zum Anwendungsbeispiel 1 wird eine Basis- und Katalysatorpaste geknetet, wobei nur je 1 Teil des Hydrophilierungsmittels aus dem Herstellungsbeispiel 2 sowohl in die Katalysator- als auch Basispaste eingebracht wird. Der 10-Sekunden-Randwinkel, der analog zum Anwendungsbeispiel 1 bestimmt wird, beträgt 35(± 3)°. Anschließend werden die Prüfkörper im handelsüblichen Desinfektionstauchbad (Impresept, ESPE) eine Stunde desinfiziert. Die Prüfkörper werden entnommen, unter fließendem kalten Wasser kurz gespült und zwei Stunden an Luft von 23°C und 50 % relativer Feuchte trocknen gelassen. Danach wird der Randwinkel erneut bestimmt. 10 Sekunden-Randwinkel beträgt wiederum 35(± 3)°.

Bei der anwendungstechnischen Prüfung analog zu dem Anwendungsbeispiel 1 erhält man nach dem Ausgießen einer Abformung mit Gipsaufschlämmung ein Gipsmodell mit einer blasenfreien, einwandfreien Oberfläche. Die Dimensionsstabilität nach ADA 19 wird zu -0,2 % bestimmt. Die Lagerstabilität wird analog zum Anwendungsbeispiel 1 überprüft, wobei sich nach einer Einlagerung von 14 Tagen bei 70°C keine Veränderung des Abbindeverlaufs gelagerter im Vergleich zu nicht gealterten Pasten ergibt.

**Herstellungsbeispiel 3**

$$CH_2=CH-(CH_2)_2-CH=CH_2 + 2\ H-SiMe_2Cl \rightarrow \rightarrow HMe_2Si-(CH_2)_6-SiMe_2H$$

82,1 g (1,0 M) 1,6-Hexadien werden in 100 ml aromatischem Lösungsmittel mit Pt-Katalysator versetzt und bei 70°C mit 208,2 g (2,20 M) Dimethylchlorsilan hydrosilyliert. Die Reaktionsmischung wird über $Al_2O_3$ gesäult, auf die Hälfte des Volumens eingeengt und mit 18,5 g (0,5 M) $LiAlH_4$-Lösung in THF hydriert. Nach hydrolytischer Aufarbeitung wird von der organischen Phase das Lösungsmittel abdestilliert. Zurück bleibt ein klares farbloses Öl. Ausbeute an obigem Silan: 145,8 g - 72 %.

$$HMe_2Si-(CH_2)_6-SiMe_2H + 2\ MeO-(EO)_{\approx 8}-CH_2CH=CH_2 \rightarrow$$

$$MeO-(EO)_{\approx 8}-(CH_2)_3-Me_2Si-(CH_2)_6-SiMe_2-(CH_3)_3-O-(EO)_{\approx 8}-Me$$

30 g (0,15 M) des erhaltenen Silans werden in 50 ml Toluol mit 86,5 g (0,22 M) Polyethylenglykolmethylallylether versetzt und mittels Platin-Katalysator hydrosilyliert. Das Produkt wird mittels einer $Al_2O_3$-Säule gereinigt und von Lösungsmittel befreit. Ausbeute an erhaltenem Polyethercarbosilan: 98 g - 90 %.

**Herstellungsbeispiel 4**

Polyethylenglykol ($M_w$ = 400 g/Mol) wird nach Vorschrift (M. Galin, A. Mathis, Macromol. 15 (1981) 677) allyliert.

$$CH_2=CH-CH_2-O-(CH_2-CH_2-O)_{\approx 9}-CH_2-CH=CH_2 + 2[(CH_3)_3SiCH_2]_2Si(CH_3)H$$

$$\rightarrow [(CH_3)_3SiCH_2]_2Si(CH_3)-(CH_2)_3-O-(CH_2-CH_2-O)_{\approx 9}-(CH_2)_3Si(CH_3)[CH_2Si(CH_3)_3]_2$$

30 g (0,063 M) des erhaltenen PEG-400 Diallylethers werden mit 32,7 g (0,15 M) Bis(trimethylsilylmethyl)methylsilan in einem aromatischen Lösungsmittel unter Platinkatalyse hydrosilyliert. Die Reaktion dauert bei 100°C 80 Stunden. Ausbeute an erhaltenem Polyethercarhosilan: 47 g - 81 %.

**Patentansprüche**

1. Zahnabdruckmasse, enthaltend 0,1 bis 10 Gew.-%, bezogen auf das Gesamtgewicht der Masse, eines Polyethercarbosilans als Hydrophilierungsmittel.

2. Zahnabdruckmasse nach Anspruch 1, dadurch gekennzeichnet, daß das Polyethercarbosilan die allgemeine Formel besitzt

$$Q - P - (OC_nH_{2n})_x - OT \quad (I)$$

in welcher bedeuten

Q $R_3Si$ -
oder

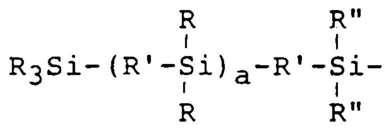

wobei jedes R im Molekül gleich oder verschieden sein kann und einen aliphatischen $C_1$-$C_{18}$-, einen cycloaliphatischen $C_6$-$C_{12}$- oder einen aromatischen $C_6$-$C_{12}$-Kohlenwasserstoffrest, die gegebenenfalls durch Halogenatome substituiert sein können, bedeutet,

R' eine $C_1$-$C_{14}$-Alkylengruppe ist,
R" im Falle von a ≠ 0 R ist oder im Falle von a = 0 R oder $R_3SiR'$ ist, und a = 0-2 bedeutet;

P eine $C_2$-$C_{18}$-Alkylengruppe, vorzugsweise eine $C_2$-$C_{14}$-Alkylengruppe oder S-R"', wobei S eine $C_2$-$C_{18}$-Alkylengruppe und R"' eine funktionelle Gruppe aus folgender Liste darstellt:

-NHC(O)-, $-NHC(O)(CH_2)_{n-1}-$, -NHC(O)C(O)-,
-NHC(O) $(CH_2)_vC(O)$-, -OC(O)-, s
$-OC(O)(CH_2)_{n-1}-$, -OC(O)C(O)-, $-OC(O)(CH_2)_vC(O)-$,
$-OCH_2CH(OH)CH_2OC(O)(CH_2)_{n-1}-$,
$-OCH_2CH(OH)CH_2OC(O)(CH_2)_vC(O)-$

mit v = 1-12;

T gleich H oder einen $C_1$-$C_4$-Alkylrest oder einen $C_1$-$C_4$-Acylrest;

x eine Zahl von 1 bis 200; und

n eine mittlere Zahl von 1 bis 6, vorzugsweise 1 bis 4.

**3.** Zahnabdruckmasse nach Anspruch 2, dadurch gekennzeichnet, daß das Polyethercarbosilan ausgewählt ist aus den folgenden Verbindungen:

$$Et_3Si\text{-}(CH_2)_3\text{-}O\text{-}(C_2H_4O)_y\text{-}CH_3$$

$$Et_3Si\text{-}CH_2\text{-}CH_2\text{-}O\text{-}(C_2H_4O)_y\text{-}CH_3$$

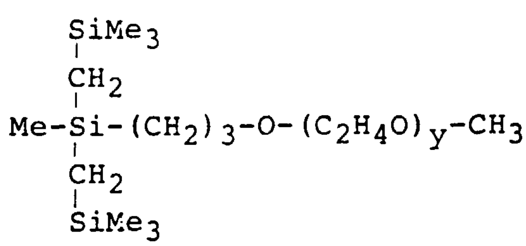

$$(Me_3Si\text{-}CH_2)_3Si\text{-}(CH_2)_3\text{-}O\text{-}(C_2H_4O)_y\text{-}CH_3$$

$$Me_3Si\text{-}CH_2\text{-}SiMe_2\text{-}(CH_2)_3\text{-}O\text{-}(C_2H_4O)_y\text{-}CH_3$$

$Me_3Si-(CH_2)_3-O-(C_2H_4O)_y-CH_3$

$Me_3Si-CH_2-CH_2-O-(C_2H_4O)_y-CH_3$

$Ph_3Si-(CH_2)_3-O-(C_2H_4O)_y-CH_3$, Ph = Phenyl

$Ph_3Si-CH_2-CH_2-O-(C_2H_4O)_y-CH_3$, Ph = Phenyl

$Cy_3Si-(CH_2)_3-O-(C_2H_4O)_y-CH_3$, Cy = Cyclohexyl

$Cy_3Si-CH_2-CH_2-O-(C_2H_4O)_y-CH_3$, Cy = Cyclohexyl

$(C_6H_{13})_3Si-(CH_2)_3-O-(C_2H_4O)_y-CH_3$

$(C_6H_{13})_3Si-CH_2-CH_2-O-(C_2H_4O)_y-CH_3$

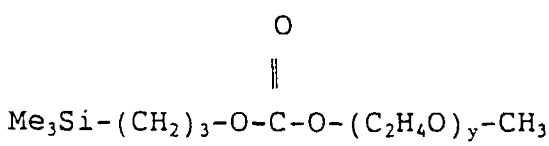

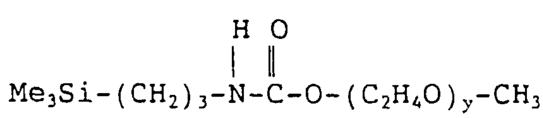

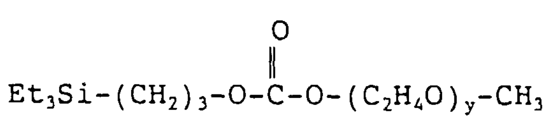

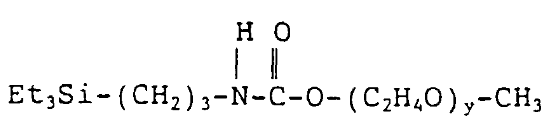

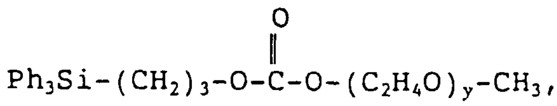, Ph = Phenyl

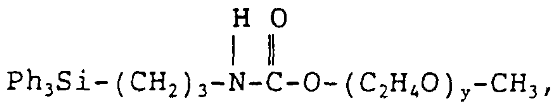, Ph = Phenyl

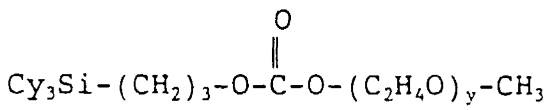, Cy = Cyclohexyl

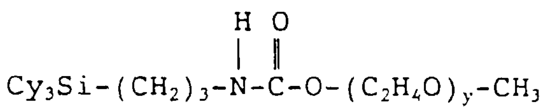, Cy = Cyclohexyl

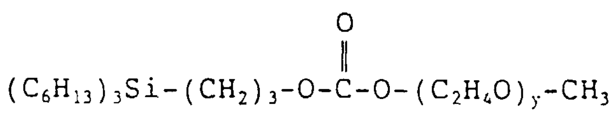

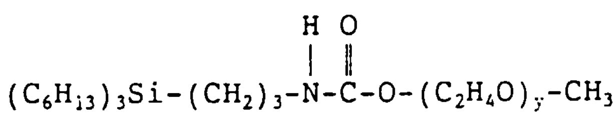

bei denen y der Beziehung genügt:
$1 \leq y \leq 20$ oder vorzugsweise $1 \leq y \leq 10$.

**4.** Zahnabdruckmasse nach Anspruch 1, dadurch gekennzeichnet, daß das Polyethercarbosilan die allgemeine Formel besitzt:

$$Q'\text{-}(P\text{-}(OC_nH_{2n})_x\text{-}OT)_2 \quad \text{(II)}$$

worin bedeuten:

Q' $-SiR_2-X-SiR_2-$;

X einen zweiwertigen Kohlenwasserstoffrest, d.h. ein $C_1$-$C_{18}$-Alkylen-, $C_6$-$C_{20}$-Arylen-, $C_5$-$C_{15}$-Cycloalkylen und $C_5$-$C_{15}$-Polycycloalkylenrest, der gegebenenfalls sauerstoffhaltige Gruppen tragen kann, und wobei

alle weiteren Symbole die gleiche Bedeutung wie in Anspruch 2 haben.

5. Zahnabdruckmasse nach Anspruch 4, dadurch gekennzeichnet, daß das Polyethercarbosilan ausgewählt ist aus den folgenden Verbindungen:

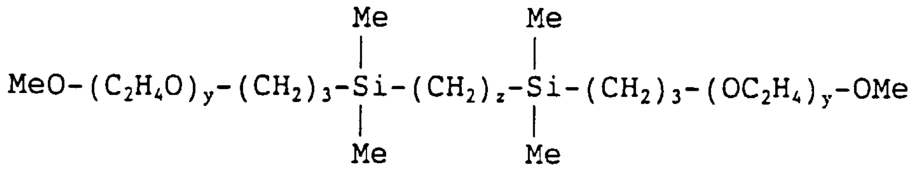

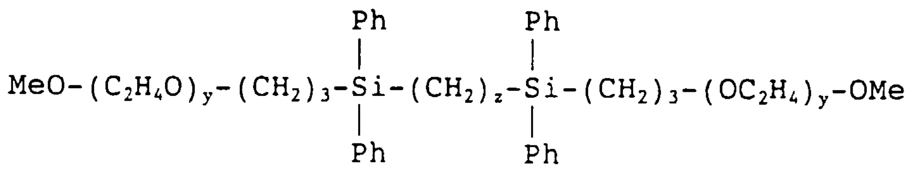

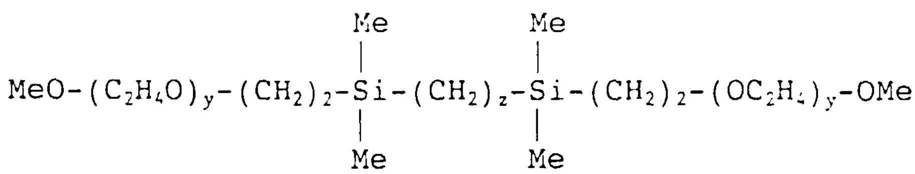

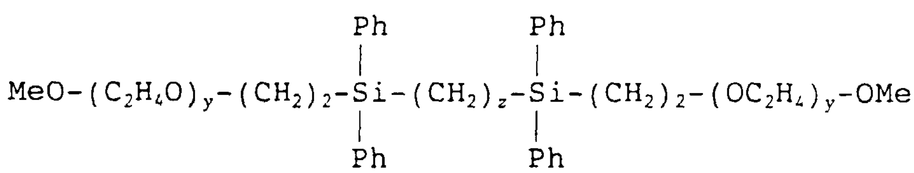

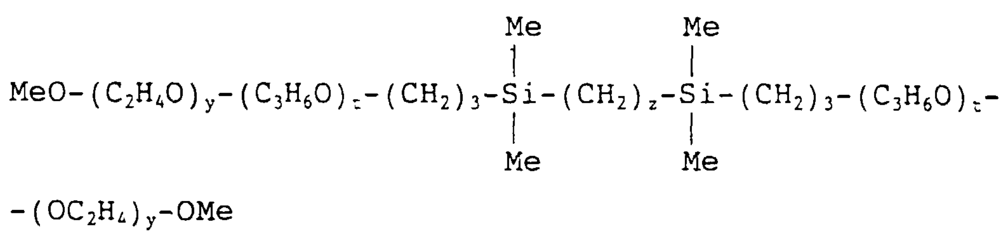

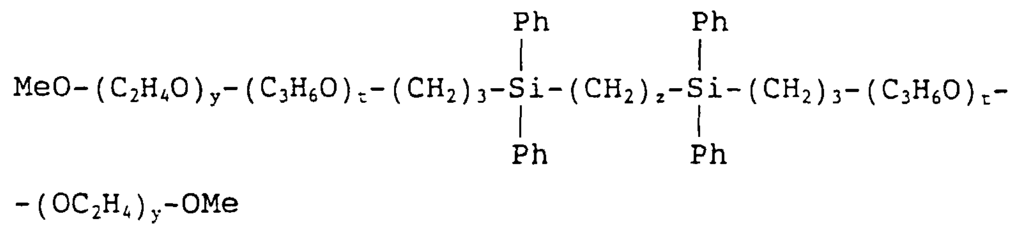

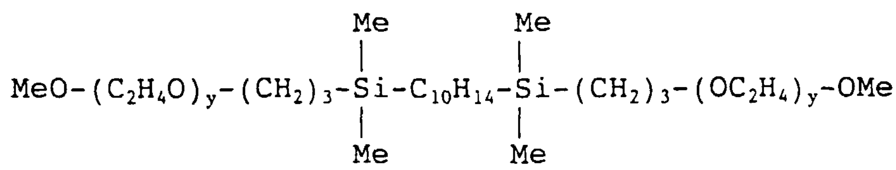

$C_{10}H_{14}$ = Tricyclo[5.2.1.0$^{2,6}$]dec-3(4),8(9)-ylen

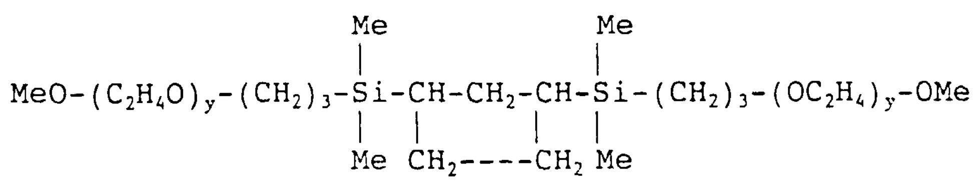

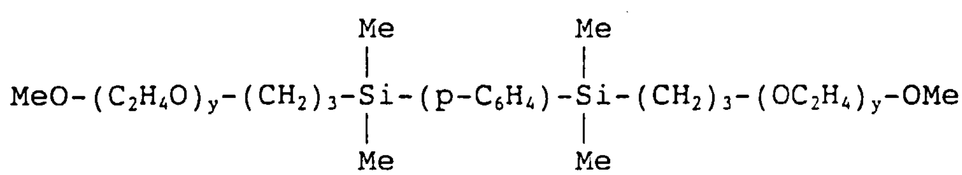

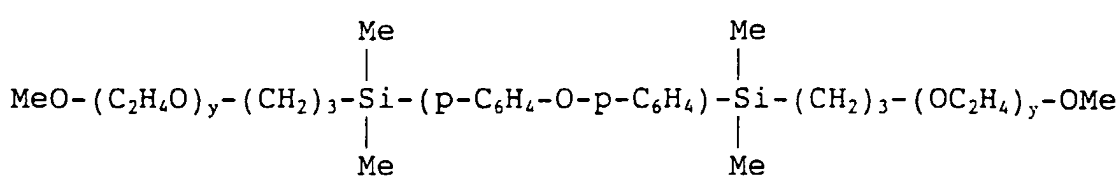

bei denen y der Beziehung genügt:

$1 \leq y \leq 20$ oder vorzugsweise $1 \leq y \leq 10$,

und z der Beziehung genügt:

$2 \leq z \leq 8$ oder vorzugsweise $2 \leq z \leq 6$;

und t der Beziehung genügt:

$0{,}1 \cdot y \leq t \leq y$ oder vorzugsweise $0{,}15 \cdot y \leq t \leq 0{,}55 \cdot y$.

**6.** Zahnabdruckmasse nach Anspruch 1, dadurch gekennzeichnet, daß das Polyethercarbosilan die allgemeine For-

mel besitzt:

$$Q\text{-}P\text{-}(OC_nH_{2n})_x\text{-}OP\text{-}Q \qquad (III)$$

worin alle Symbole die gleiche Bedeutung haben wie in Anspruch 2.

**7.** Zahnabdruckmasse nach Anspruch 6, dadurch gekennzeichnet, daß das Polyethercarbosilan ausgewählt ist aus den folgenden Verbindungen:

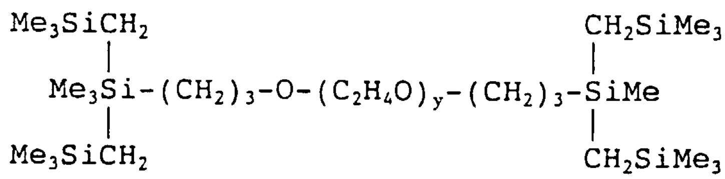

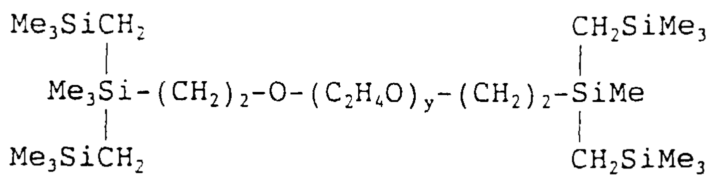

$(Cy)_2MeSi\text{-}(CH_2)_3\text{-}O\text{-}(C_2H_4O)_y\text{-}(CH_2)_3\text{-}SiMe(Cy)_2$, Cy = Cyclohexyl

$(Cy)_2MeSi\text{-}(CH_2)_2\text{-}O\text{-}(C_2H_4O)_y\text{-}(CH_2)_2\text{-}SiMe(Cy)_2$, Cy = Cyclohexyl

bei denen y der Beziehung genügt:
$1 \leq y \leq 30$ oder vorzugsweise $2 \leq y \leq 20$.

**8.** Zahnabdruckmasse nach Anspruch 1, dadurch gekennzeichnet, daß das Polyethercarbosilan die allgemeine Formel besitzt:

$$Q\text{-}P'\text{-}(OC_nH_{2n})_x\text{-}OT \qquad (IV)$$

worin bedeuten:

P' 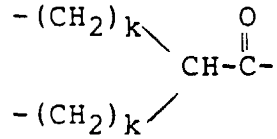

oder

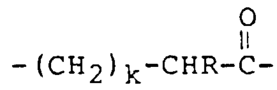

oder

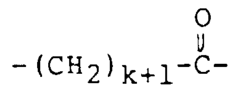

wobei k 1-8 ist; und wobei
alle weiteren Symbole die gleiche Bedeutung wie in Anspruch 2 haben.

**9.** Zahnabdruckmasse nach Anspruch 8, dadurch gekennzeichnet, daß das Polyethercarbosilan ausgewählt ist aus den folgenden Verbindungen:

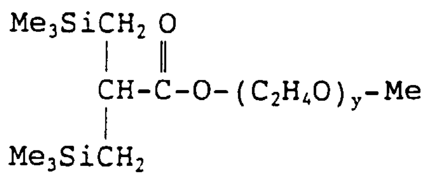

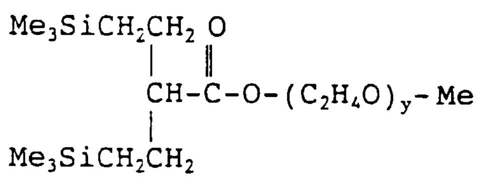

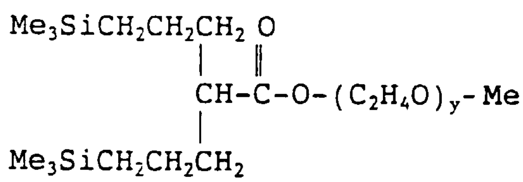

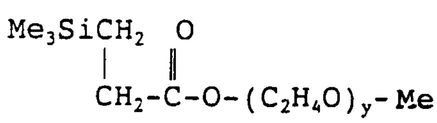

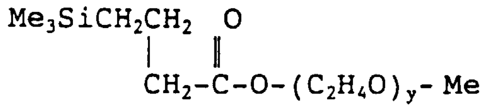

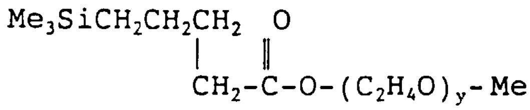

bei denen y der Beziehung genügt:

$1 \leq y \leq 20$ oder vorzugsweise $1 \leq y \leq 10$.

**10.** Zahnabdruckmasse nach Anspruch 1, dadurch gekennzeichnet, daß das Polyethercarbosilan die allgemeine Formel besitzt:

$$Q''[-P-(OC_nH_{2n})_xOT]_o \qquad (V)$$

worin bedeutet:

Q" ein oligomeres oder polymeres Carbosilan, wobei mindestens ein Siliciumatom, vorzugsweise aber 20 - 70 % der Siliciumatome mit dem Rest $-P-(OC_nH_{2n})_xOT$ substituiert sind und damit $o \geq 1$ ist; und wobei

alle weiteren Symbole die gleiche Bedeutung wie in Anspruch 2 besitzen.

**11.** Zahnabdruckmasse nach Anspruch 10, dadurch gekennzeichnet, daß das Polyethercarbosilan ausgewählt ist aus den folgenden Verbindungen

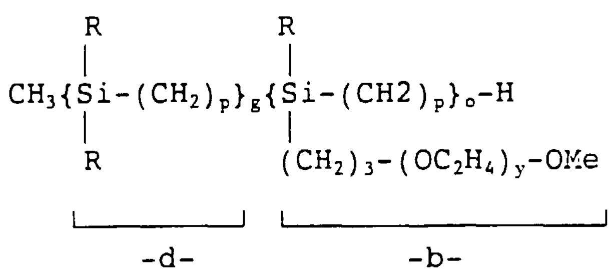

bei denen y der Beziehung genügt:

$1 \leq y \leq 20$ oder vorzugsweise $1 \leq y \leq 10$;

und p gleich 2 oder 3 sein kann;

o vorzugsweise 3-1000 ist und g insbesondere der Beziehung genügt:

$4 \cdot o \geq g \geq 0{,}42 \cdot o$, und

R vorzugsweise Methyl, Ethyl oder Phenyl bedeutet.

**12.** Zahnabdruckmasse nach den Ansprüchen 1 bis 11, dadurch gekennzeichnet, daß die Basis der Zahnabdruckmasse von additions- oder kondensationsvernetzenden Silikonen, Polyethersilikonen oder Polyethern gebildet wird.

**13.** Verwendung eines Polyethercarbosilans zur Hydrophilierung von Zahnabdruckmassen.

**14.** Verfahren zur Herstellung von Zahnabformungen, dadurch gekennzeichnet, daß man eine mit einem Polyethercarbosilan hydrophilierte Zahnabdruckmasse verwendet.

## Claims

**1.** Dental impression compound, containing 0.1 to 10 wt.-%, relative to the total weight of the compound, of a polyether carbosilane as hydrophilation agent.

**2.** Dental impression compound according to claim 1, characterized in that the polyether carbosilane has the general formula

$$Q - P - (OC_nH_{2n})_x - OT \qquad (I)$$

in which:

Q stands for $R_3Si$ -
or

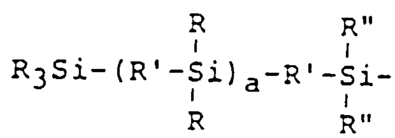

where every R in the molecule can be the same or different and stands for an aliphatic $C_1$-$C_{18}$, a cycloaliphatic $C_6$-$C_{12}$ or an aromatic $C_6$-$C_{12}$ hydrocarbon radical, which can optionally be substituted by halogen atoms,

R' is a $C_1$-$C_{14}$ alkylene group,
R''' is R in the case of $a \neq 0$ or is R or $R_3SiR'$ in the case of a = 0, and a = 0-2;

P stands for a $C_2$-$C_{18}$ alkylene group, preferably a $C_2$-$C_{14}$ alkylene group or S-R''', where S represents a $C_2$-$C_{18}$ alkylene group and R''' a functional group from the following list:

$-NHC(O)-$, $-NHC(O)(CH_2)_{n-1}-$, $-NHC(O)C(O)-$,
$-NHC(O)(CH_2)_vC(O)-$, $-OC(O)-$,
$-OC(O)(CH_2)_{n-1}-$, $-OC(O)C(O)-$, $-OC(O)(CH_2)_vC(O)-$,
$-OCH_2CH(OH)CH_2OC(O)(CH_2)_{n-1}-$,
$-OCH_2CH(OH)CH_2OC(O)(CH_2)_vC(O)-$

with v = 1-12;

T is the same as H or stands for a $C_1$-$C_4$ alkyl radical or a $C_1$-$C_4$ acyl radical;

x stands for a number from 1 to 200; and

n stands for an average number from 1 to 6, preferably 1 to 4.

**3.** Dental impression compound according to claim 2, characterized in that the polyether carbosilane is selected from the following compounds:

$Et_3Si-(CH_2)_3-O-(C_2H_4O)_y-CH_3$

$Et_3Si-CH_2-CH_2-O-(C_2H_4O)_y-CH_3$

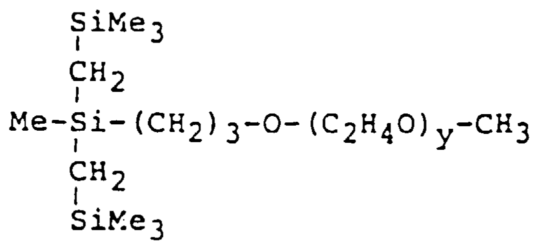

$(Me_3Si-CH_2)_3Si-(CH_2)_3-O-(C_2H_4O)_y-CH_3$

$Me_3Si-CH_2-SiMe_2-(CH_2)_3-O-(C_2H_4O)_y-CH_3$

$Me_3Si-(CH_2)_3-O-(C_2H_4O)_y-CH_3$

$Me_3Si-CH_2-CH_2-O-(C_2H_4O)_y-CH_3$

$Ph_3Si-(CH_2)_3-O-(C_2H_4O)_y-CH_3$, Ph = Phenyl

$Ph_3Si-CH_2-CH_2-O-(C_2H_4O)_y-CH_3$, Ph = Phenyl

$Cy_3Si-(CH_2)_3-O-(C_2H_4O)_y-CH_3$, Cy = Cyclohexyl

$Cy_3Si-CH_2-CH_2-O-(C_2H_4O)_y-CH_3$, Cy = Cyclohexyl

$(C_6H_{13})_3Si-(CH_2)_3-O-(C_2H_4O)_y-CH_3$

$(C_6H_{13})_3Si-CH_2-CH_2-O-(C_2H_4O)_y-CH_3$

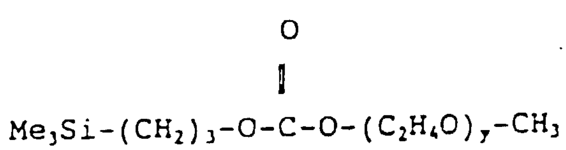

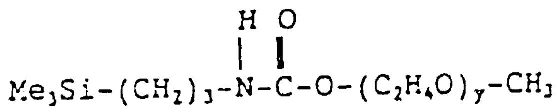

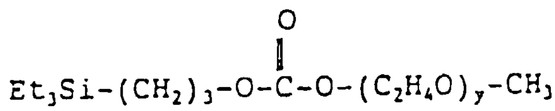

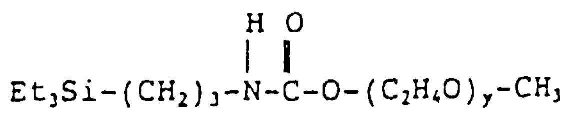

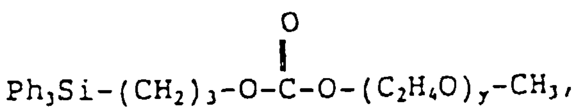
Ph = Phenyl

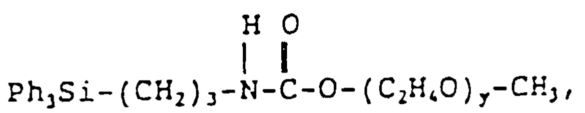
Ph = Phenyl

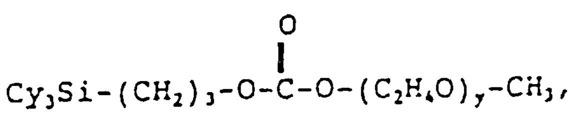
Cy = Cyclohexyl

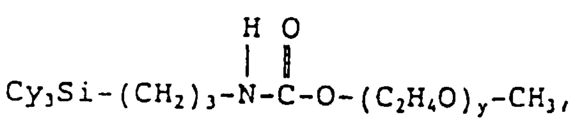
Cy = Cyclohexyl

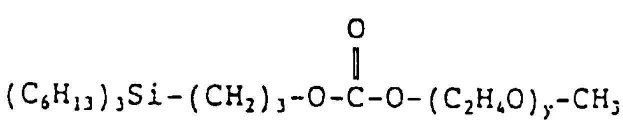

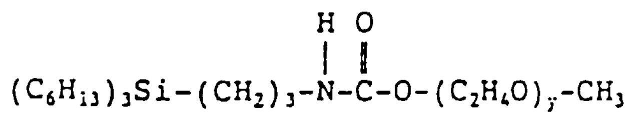

in which y conforms to the relation:
$1 \leq y \leq 20$ or preferably $1 \leq y \leq 10$.

**4.** Dental impression compound according to claim 1, characterized in that the polyether carbosilane has the general formula:

$$Q'\text{-}(P\text{-}(OC_nH_{2n})_x\text{-}OT)_2 \qquad (II)$$

in which:

Q' stands for $-SiR_2-X-SiR_2-$;

X stands for a divalent hydrocarbon radical, i.e. a $C_1$-$C_{18}$ alkylene, $C_6$-$C_{20}$ arylene, $C_5$-$C_{15}$ cycloalkylene and $C_5$-$C_{15}$ polycycloalkylene radical, which can optionally bear oxygen-containing groups, and where

all other symbols have the same meaning as in claim 2.

**5.** Dental impression compound according to claim 4, characterized in that the polyether carbosilane is selected from the following compounds:

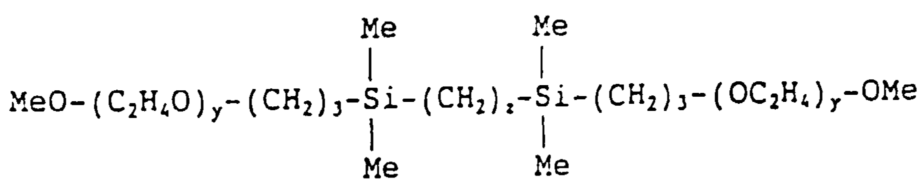

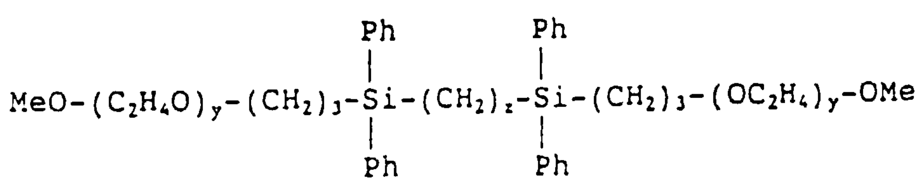

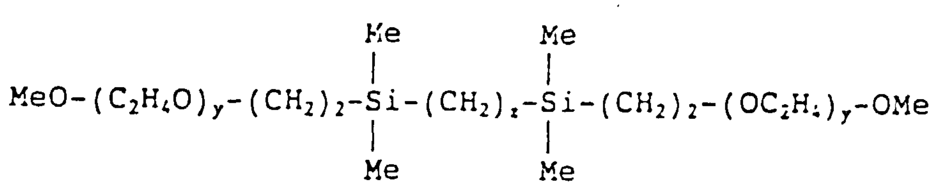

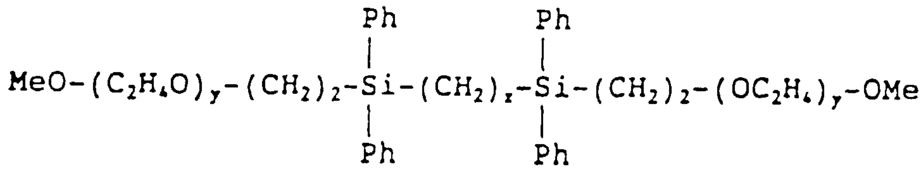

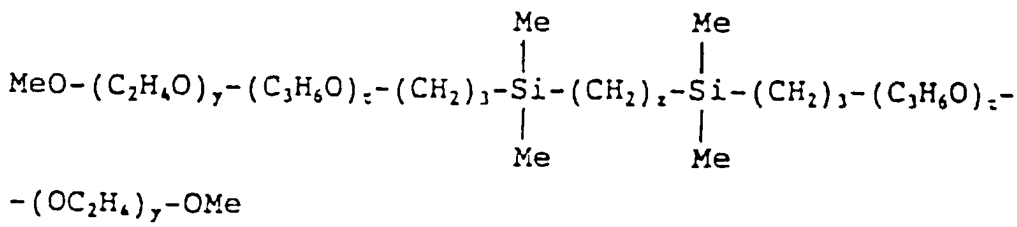

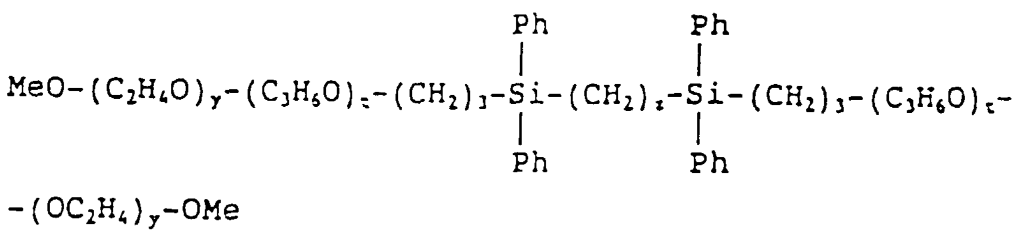

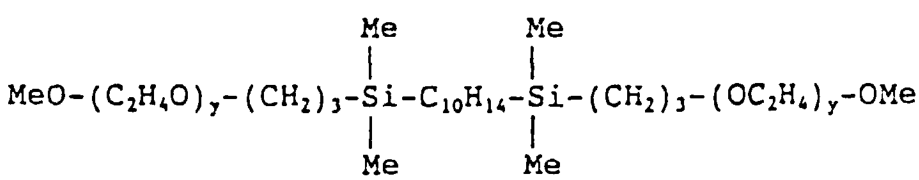

$C_{10}H_{14}$ = Tricyclo[5.2.1.$0^{2,6}$]dec-3(4),8(9)-ylen

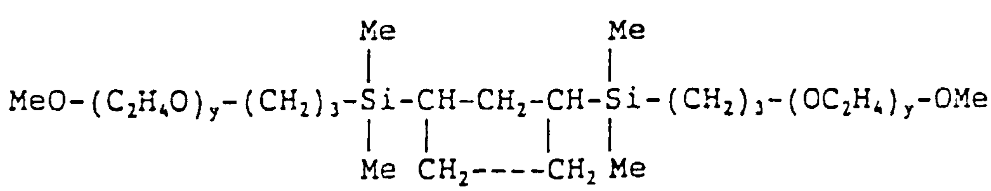

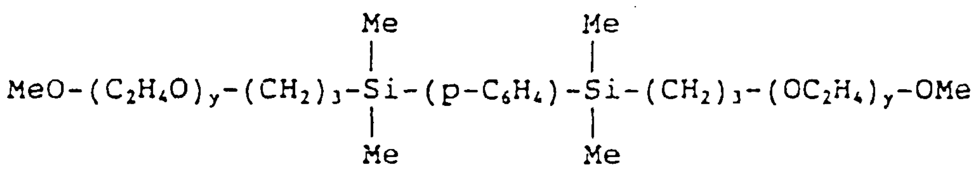

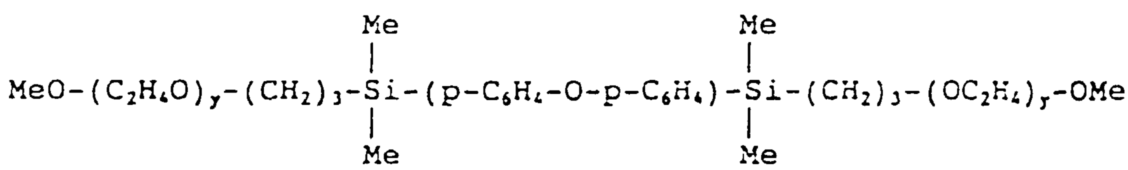

in which y conforms to the relation:

$1 \leq y \leq 20$ or preferably $1 \leq y \leq 10$,

and z conforms to the relation:

$2 \leq z \leq 8$ or preferably $2 \leq z \leq 6$;

and t conforms to the relation:

$0.1 \cdot y \leq t \leq y$ or preferably $0.15 \cdot y \leq t \leq 0.55 \cdot y$.

**6.** Dental impression compound according to claim 1, characterized in that the polyether carbosilane has the general formula:

$$Q\text{-}P\text{-}(OC_nH_{2n})_x\text{-}OP\text{-}Q \qquad \text{(III)}$$

in which all symbols have the same meaning as in claim 2.

**7.** Dental impression compound according to claim 6, characterized in that the polyether carbosilane is selected from the following compounds:

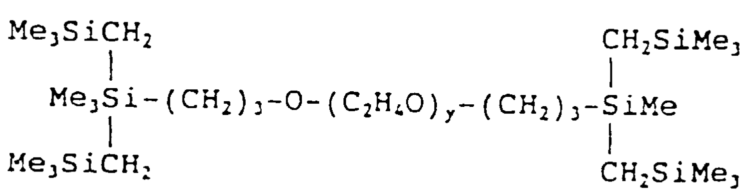

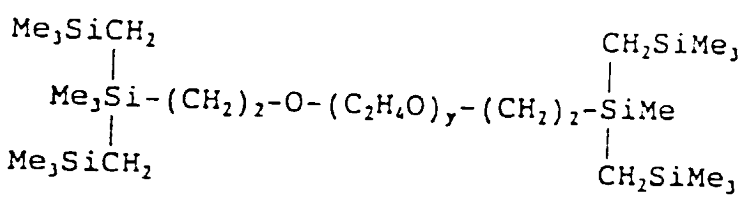

$(Cy)_2MeSi\text{-}(CH_2)_3\text{-}O\text{-}(C_2H_4O)_y\text{-}(CH_2)_3\text{-}SiMe(Cy)_2$, Cy = Cyclohexyl

$$(Cy)_2MeSi\text{-}(CH_2)_2\text{-}O\text{-}(C_2H_4O)_y\text{-}(CH_2)_2\text{-}SiMe(Cy)_2,\ Cy = \text{Cyclohexyl}$$

in which y conforms to the relation:
$1 \leq y \leq 30$ or preferably $2 \leq y \leq 20$.

**8.** Dental impression compound according to claim 1, characterized in that the polyether carbosilane has the general formula:

$$Q\text{-}P'\text{-}(OC_nH_{2n})_x\text{-}OT \qquad (IV)$$

in which:
P' stands for

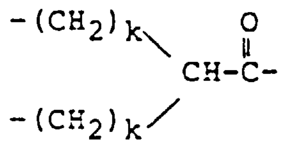

or

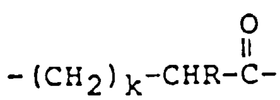

or

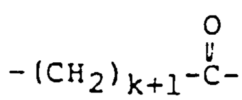

where k is 1-8; and where
all other symbols have the same meaning as in claim 2.

**9.** Dental impression compound according to claim 8, characterized in that the polyether carbosilane is selected from the following compounds:

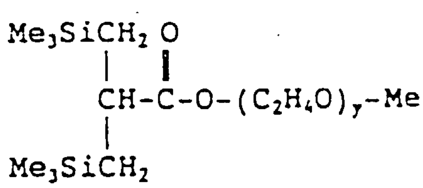

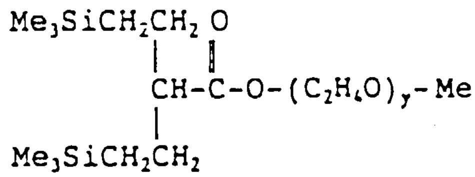

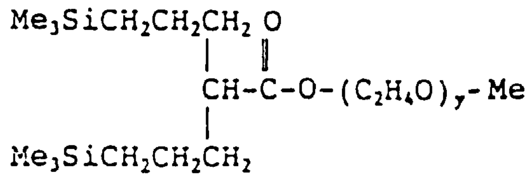

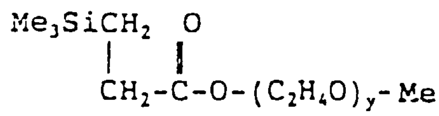

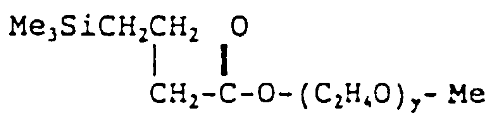

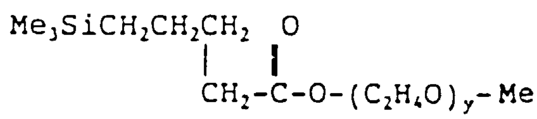

in which y conforms to the relation:

$1 \leq y \leq 20$ or preferably $1 \leq y \leq 10$.

**10.** Dental impression compound according to claim 1, characterized in that the polyether carbosilane has the general formula:

$$Q''[-P-(OC_nH_{2n})_xOT]_o \qquad (V)$$

in which:

Q'' stands for an oligomeric or polymeric carbosilane, where at least one silicon atom, but preferably 20-70 % of the silicon atoms, are substituted by the radical $-P-(OC_nH_{2n})_XOT)$ and thus o is ≥ 1 ; and where all other symbols have the same meaning as in claim 2.

**11.** Dental impression compound according to claim 10, characterized in that the polyether carbosilane is selected from the following compounds

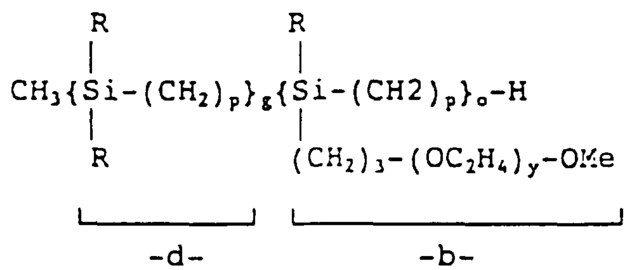

in which y conforms to the relation:

$1 \leq y \leq 20$ or preferably $1 \leq y \leq 10$; and p can equal 2 or 3;

o is preferably 3-1000 and g in particular conforms to the relation:

$4 \cdot o \geq g \geq 0.42 \cdot o$, and

R is preferably methyl, ethyl or phenyl.

**12.** Dental impression compound according to claims 1 to 11, characterized in that the basis of the dental impression compound is formed from addition- or condensation- crosslinking silicones, polyether silicones or polyethers.

**13.** Use of a polyether carbosilane for the hydrophilation of dental impression compounds.

**14.** Process for the production of dental mouldings, characterized in that a dental impression compound hydrophilated with a polyether carbosilane is used.

## Revendications

**1.** Composition pour empreinte dentaire, contenant de 0,1 à 10 % en poids, par rapport au poids total de la composition, d'un polyéthercarbosilane en tant qu'agent d'hydrophilisation.

**2.** Composition pour empreinte dentaire selon la revendication 1, caractérisée en ce que le polyéthercarbosilane a la formule générale

$$Q - P - (OC_nH_{2n})_x - OT \qquad (I)$$

dans laquelle

Q est $R_3Si$-
ou

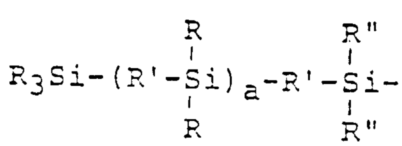

où les radicaux R de la molécule peuvent être identiques ou différents et représentent chacun un radical hydrocarboné aliphatique en $C_1$-$C_{18}$, cycloaliphatique en $C_6$-$C_{12}$ ou aromatique en $C_6$-$C_{12}$, qui peut être éventuellement substitué par des atomes d'halogène,

R' est un groupe alkylène en $C_1$-$C_{14}$,
R", quand a ≠ 0, est R, ou encore, quand a = 0, est R ou $R_3SiR'$, et a = 0-2;
P est un groupe alkylène en $C_2$-$C_{18}$, de préférence un groupe alkylène en $C_2$-$C_{14}$ ou S-R"', où S est un groupe alkylène en $C_2$-$C_{18}$ et R"' est un groupe fonctionnel choisi dans la liste suivante :

$-NHC(O)-$, $-NHC(O)(CH_2)_{n-1}-$, $-NHC(O)C(O)-$,
$-NHC(O)(CH_2)_vC(O)-$, $-OC(O)-$,
$-OC(O)(CH_2)_{n-1}-$, $-OC(O)C(O)-$, $-OC(O)(CH_2)_vC(O)-$,
$-OCH_2CH(OH)CH_2OC(O)(CH_2)_{n-1}-$,
$-OCH_2CH(OH)CH_2OC(O)(CH_2)_vC(O)-$

avec v = 1-12 ;
T est H ou un radical alkyle en $C_1$-$C_4$ ou un radical acyle en $C_1$-$C_4$ ;
x est un nombre de 1 à 200 ; et
n vaut en moyenne de 1 à 6, de préférence de 1 à 4.

**3.** Composition pour empreinte dentaire selon la revendication 2, caractérisée en ce que le polyéthercarbosilane est choisi parmi les composés suivants :

$$Et_3Si-(CH_2)_3-O-(C_2H_4O)_y-CH_3$$

$$Et_3Si-CH_2-CH_2-O-(C_2H_4O)_y-CH_3$$

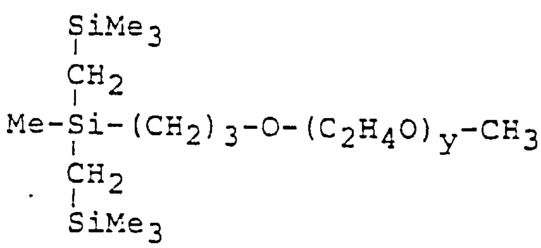

$$(Me_3Si-CH_2)_3Si-(CH_2)_3-O-(C_2H_4O)_y-CH_3$$

$$Me_3Si-CH_2-SiMe_2-(CH_2)_3-O-(C_2H_4O)_y-CH_3$$

$$Me_3Si-(CH_2)_3-O-(C_2H_4O)_y-CH_3$$

$$Me_3Si-CH_2-CH_2-O-(C_2H_4O)_y-CH_3$$

$Ph_3Si-(CH_2)_3-O-(C_2H_4O)_y-CH_3$, Ph = Phenyl

$Ph_3Si-CH_2-CH_2-O-(C_2H_4O)_y-CH_3$, Ph = Phenyl

$Cy_3Si-(CH_2)_3-O-(C_2H_4O)_y-CH_3$, Cy = Cyclohexyl

$Cy_3Si-CH_2-CH_2-O-(C_2H_4O)_y-CH_3$, Cy = Cyclohexyl

$(C_6H_{13})_3Si\text{-}(CH_2)_3\text{-}O\text{-}(C_2H_4O)_y\text{-}CH_3$

$(C_6H_{13})_3Si\text{-}CH_2\text{-}CH_2\text{-}O\text{-}(C_2H_4O)_y\text{-}CH_3$

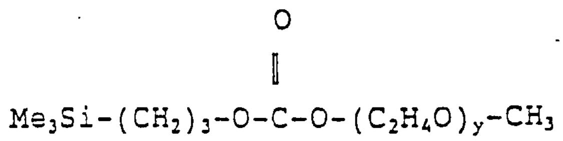

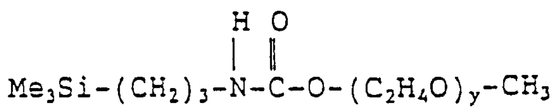

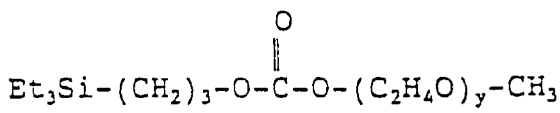

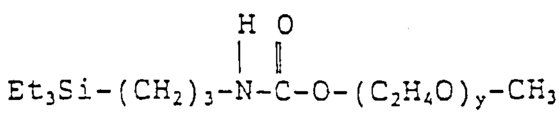

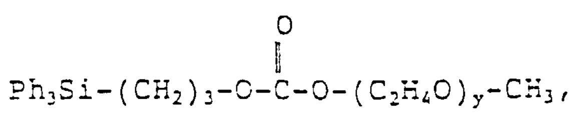 , Ph = Phenyl

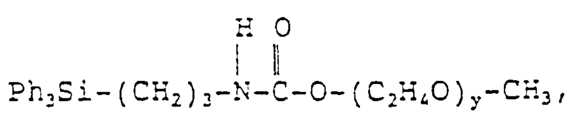 , Ph = Phenyl

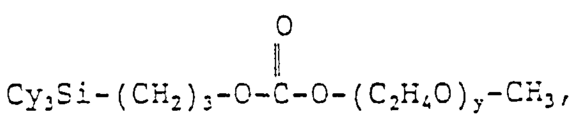 , Cy = Cyclohexyl

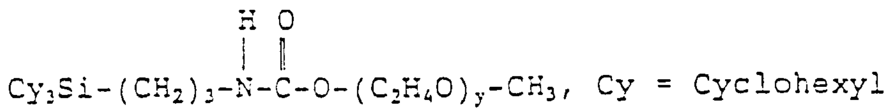

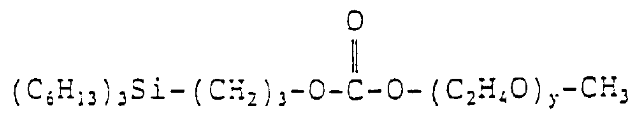

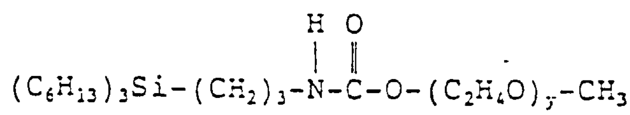

dans lesquels y satisfait à la relation :

$1 \leq y \leq 20$ ou de préférence $1 \leq y \leq 10$.

**4.** Composition pour empreinte dentaire selon la revendication 1, caractérisée en ce que le polyéthercarbosilane a la formule générale :

$$Q'\text{-}(P\text{-}(OC_nH_{2n})_x\text{-}OT)_2 \quad (II)$$

dans laquelle :

Q' est $-SiR_2-X-SiR_2-$ ;
X est un radical hydrocarboné divalent, à savoir un radical alkylène en $C_1$-$C_{18}$, arylène en $C_6$-$C_{20}$, cycloalkylène en $C_5$-$C_{15}$ et polycycloalkylène en $C_5$-$C_{15}$, qui peut éventuellement porter des groupes oxygénés, et où

tous les autres symboles ont les mêmes significations que dans la revendication 2.

**5.** Composition pour empreinte dentaire selon la revendication 4, caractérisée en ce que le polyéthercarbosilane est choisi parmi les composés suivants :

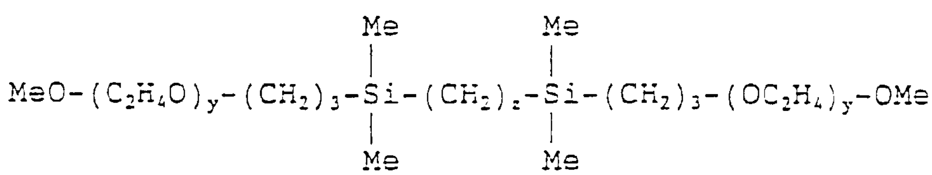

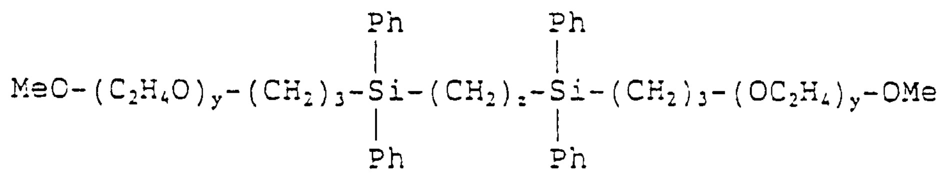

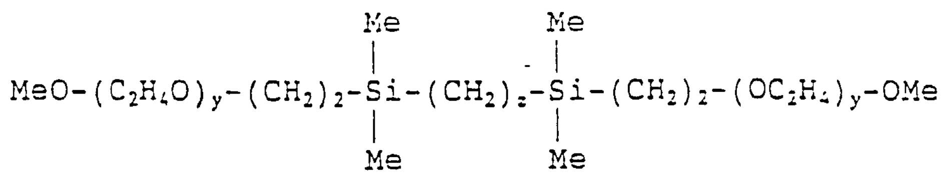

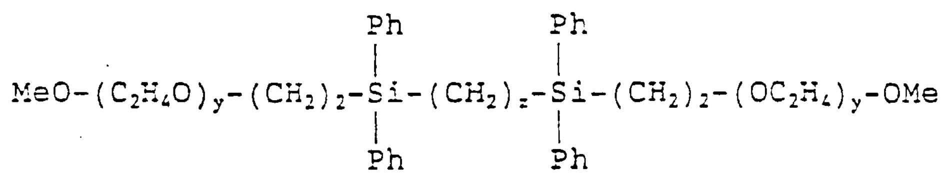

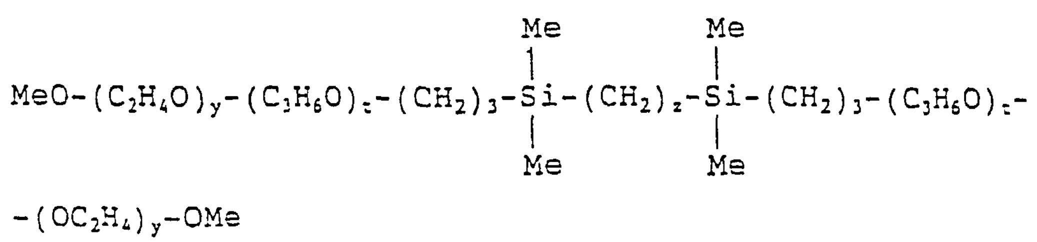

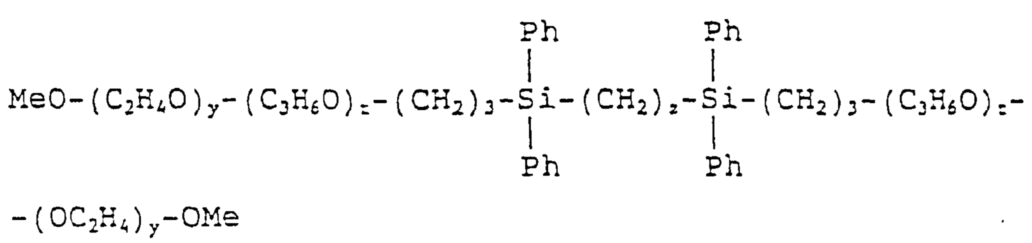

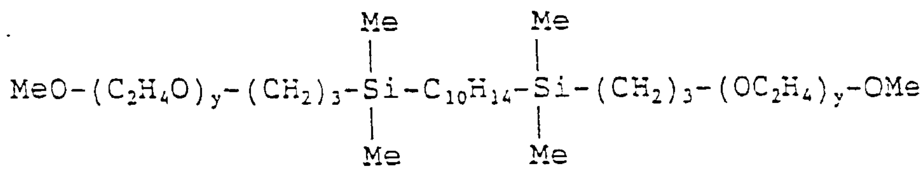

$C_{10}H_{14}$ = Tricyclo[5.2.1.$0^{2,6}$]dec-3(4),8(9)-ylen

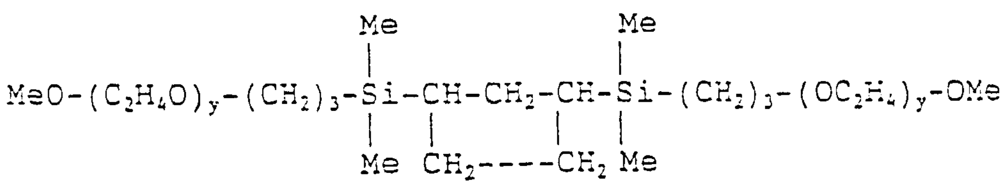

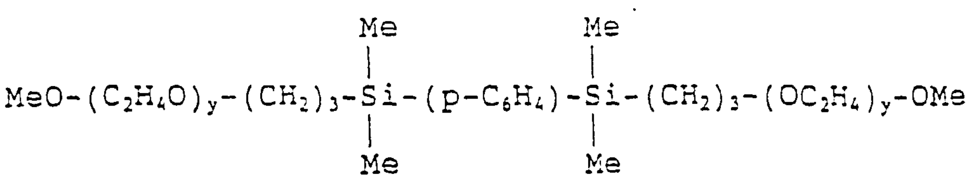

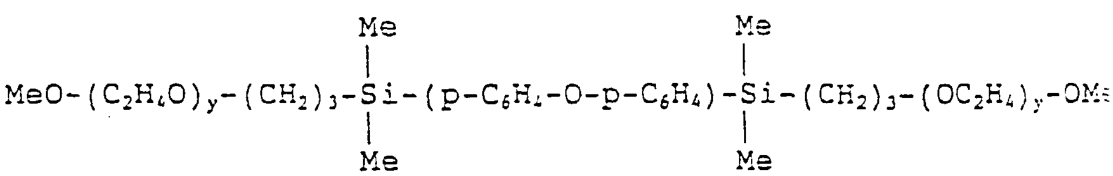

dans lesquels y satisfait à la relation :

$1 \le y \le 20$ ou de préférence $1 \le y \le 10$,

et z satisfait à la relation :

$2 \le z \le 8$ ou de préférence $2 \le z \le 6$ ;

et t satisfait à la relation :

$0,1.y \le t \le y$ ou de préférence $0,15.y \le t \le 0,55.y$.

**6.** Composition pour empreinte dentaire selon la revendication 1, caractérisée en ce que le polyéthercarbosilane a la formule générale :

$$Q\text{-}P\text{-}(OC_nH_{2n})_x\text{-}OP\text{-}Q \qquad (III)$$

dans laquelle tous les symboles ont la même signification que dans la revendication 2.

**7.** Composition pour empreinte dentaire selon la revendication 6, caractérisée en ce que le polyéthercarbosilane est choisi parmi les composés suivants :

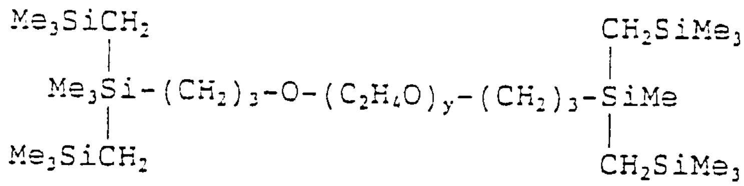

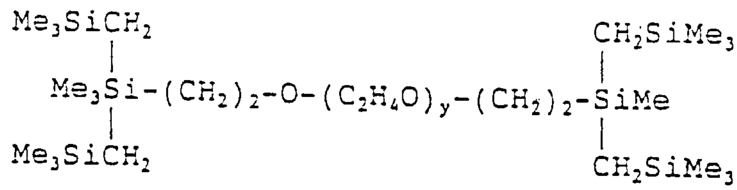

$(Cy)_2MeSi\text{-}(CH_2)_3\text{-}O\text{-}(C_2H_4O)_y\text{-}(CH_2)_3\text{-}SiMe(Cy)_2$, Cy = Cyclohexyl

$(Cy)_2MeSi\text{-}(CH_2)_2\text{-}O\text{-}(C_2H_4O)_y\text{-}(CH_2)_2\text{-}SiMe(Cy)_2$, Cy = Cyclohexyl

dans lesquels y satisfait à la relation :
$1 \leq y \leq 30$ ou de préférence $2 \leq y \leq 20$.

**8.** Composition pour empreinte dentaire selon la revendication 1, caractérisée en ce que le polyéthercarbosilane a la formule générale :

$$Q\text{-}P'\text{-}(OC_nH_{2n})_x\text{-}OT \qquad (IV)$$

dans laquelle :
P' est

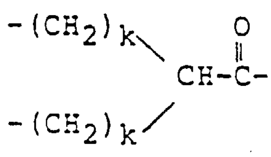

ou

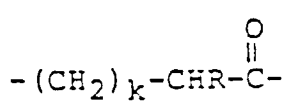

ou

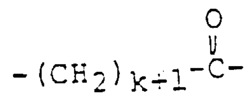

où k vaut de 1-8 ; et où tous les autres symboles ont les mêmes significations que dans la revendication 2.

**9.** Composition pour empreinte dentaire selon la revendication 8, caractérisée en ce que le polyéthercarbosilane est choisi parmi les composés suivants :

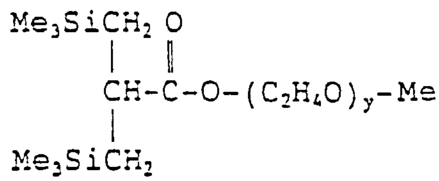

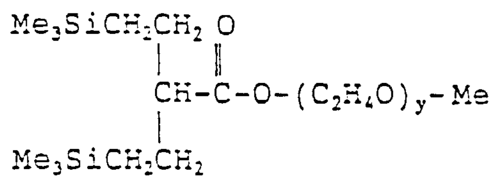

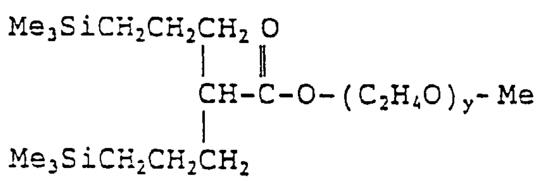

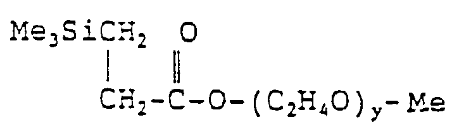

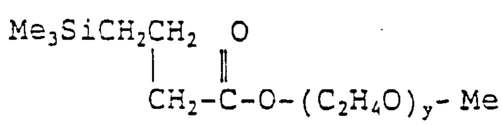

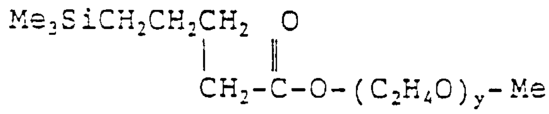

dans lesquels y satisfait à la relation :
$1 \leq y \leq 20$ ou de préférence $1 \leq y \leq 10$.

**10.** Composition pour empreinte dentaire selon la revendication 1, caractérisée en ce que le polyéthercarbosilane a la formule générale :

$$Q''[-P-(OC_nH_{2n})_xOT]_o \qquad (V)$$

dans laquelle :
Q" est un carbosilane oligomère ou polymère, au moins un atome de silicium, mais de préférence de 20 à 70 % des atomes de silicium, étant substitué par le substituant $-P-(OC_nH_{2n})_xOT$, et donc $o \geq 1$ ; et où tous les autres symboles ont les mêmes significations que dans la revendication 2.

**11.** Composition pour empreinte dentaire selon la revendication 10, caractérisée en ce que le polyéthercarbosilane est choisi parmi les composés suivants :

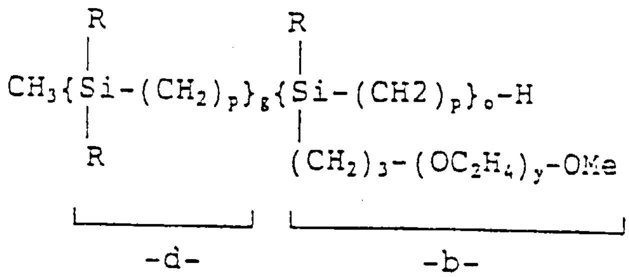

dans lesquels y satisfait à la relation :
$1 \leq y \leq 20$ ou de préférence $1 \leq y \leq 10$ ;
et p peut valoir 2 ou 3 ;
o vaut de préférence de 3 à 1000 et g satisfait en particulier à la relation :
$4.o \geq g \geq 0{,}42.o$, et
R est de préférence le groupe méthyle, éthyle ou phényle.

**12.** Composition pour empreinte dentaire selon les revendications 1 à 11, caractérisée en ce que la base de la composition pour empreinte dentaire est formée de silicones, de polyéthersilicones ou de polyéthers réticulables par addition ou par condensation.

**13.** Utilisation d'un polyéthercarbosilane pour hydrophiliser des compositions pour empreinte dentaire.

**14.** Procédé de fabrication d'empreintes dentaires, caractérisé en ce qu'on utilise une composition pour empreinte dentaire hydrophilisée avec un polyéthercarbosilane.